# EUROPEAN PATENT APPLICATION

(11) **EP 1 384 780 A1**
(43) Date of publication of application: **28.01.2004**
(21) Application number: 02027002.1
(22) Date of filing: 02.12.2002
(51) Int. Cl.: C12N 15/10

(54) **Method for generating molecules with specific properties by recombination and selection**

(30) Priority: 26.07.2002 EP 02016743
(71) Applicant: Cosmix Molecular Biologicals GmbH, 38124 Braunschweig (DE)
(72) Inventor: Collins, John, Prof. Dr., 38100 Braunschweig (DE); Szardenings, Michael, Dr., 38302 Wolfenbuettel (DE); Schürmann, Gregor, Dr., 30451 Hannover (DE); Mersmann, Michael, Dr., 38118 Braunschweig (DE)
(74) Representative: König, Gregor Sebastian, Dipl.-Biol.

(57) **Abstract**

The invention refers to a recombination process for recombining a population of heterologous polynucleotides comprising variant groups and/or regions, wherein recombination is performed within one group of polynucleotides and/or between defined regions of polynucleotides by using different restriction sites in different groups and/or regions of polynucleotides. As a result it is possible to carry out recombination with mixtures of several unrelated groups (subsets) of polynucleotides, which recombine due to the use of different restriction sites independently thus increasing diversity while maintaining structurally conserved regions in one reaction. The method can advantageously be carried out without separation of individual components or clones.

## Description

The present invention relates to an improved recombination method which allows the generation of molecules with desired traits or characteristics.

Biotech evolutionary methods are used to search for novel ligands or molecules such as polynucleotides or polypeptides using empirical procedures to select molecules with the desired characteristics, e.g. binding properties of polypeptides, from large populations of variant gene products. These methods are comparable to the process of natural evolution, since evolution includes the generation of mutation, selection of functionality over a time period and the ability of the system to self-replicate.

In order to create molecular diversity in a library in order to generate mutant molecules several methods are known as for example the error-prone polymerase chain reaction and cassette mutagenesis, in which the specific region to be optimised is replaced with a synthetically mutagenised oligonucleotide. Error-prone PCR uses low-fidelity polymerisation conditions to introduce a low level of point mutations randomly over a long sequence. This method can be used to mutagenise a mixture of fragments of unknown sequence but is limited when applied for multiple cycles due to the fact that, for example, harmful or neutral mutations accumulate. This can for example cause a protein to be immunogenic. Thus error-prone PCR can lead to undesired results and, beyond this, is too gradual to allow block changes that are required for continued sequence evolution.

Also oligonucleotide directed mutagenesis, with a short sequence being replaced by synthetically mutagenised oligonucleotides does not allow generation of combinations of distant mutations and is thus not significantly combinatorial. A high number of selection rounds and sequencing is necessary and the process thus being labour- and cost-intensive and not practicable for multiple rounds of mutagenesis.

The introduction of base changes in existing (also mutant) structures is an additive and thus a linearly increasing function. A process which relies on mutagenesis, selection and further mutagenesis also has the drawback that potentially synergistic mutations in different regions are lost since they are not selected in combination. Thus recombination processes are advantageous over processes which rely on mutagenesis in a large library. The considerable amount of extra work involved in trying to optimise a sub-optimal affinity of a lead peptide or protein, e.g. as isolated from even large libraries, but without taking the advantage of recursive or reiterative recombination or mutagenesis and recombination has been addressed before (Collins, 1997; Deshayes et al., 2002).

Recombination processes which reassort mutations accumulated in the selected population lead to an exponential increase of the combinations of mutations and thus to an increase of the number of variants in a population. Several methods are known in the state of the art which allow the production of recombinant molecules.

Known in vivo methods are based e.g. on inter-plasmidic recombination, wherein libraries of recombined polynucleotide sequences are obtained by genetic in vivo recombination of compatible or non-compatible multicopy plasmids inside suitable host cells (EP 1 138 763 A1). This can also be a site-specific recombination process. The drawback of the mentioned in vivo methods lies in the fact that molecules can only recombine in a single host cell thus severely limiting the possible diversity. Also, it is difficult to recombine at several positions within one recombination process. Further it suffers from genetic instability.

In vitro recombination ("shuffling") methods are known in the state of the art which are based on PCR and on homologous recombination. These methods are variously referred to "as sexual PCR" or "DNA shuffling", methods which aim at mimicking and accelerating evolution in the test tube to develop proteins with novel or improved characteristics (see for example EP 1 103 606 A1; EP 0911 396 A1; EP 1 138 763 A1; Stemmer et a!., 1995; Crameri and Stemmer, 1995; Schmidt-Dannert et al., 2000; Joern et al., 2002). This latter line of pursuit is often combined with reiterative rounds of mutation, recombination and selection. The initial procedures described, involve randomized partial cleavage of populations of DNA molecules with unspecific endonucleases such as DNAsel and DNA amplification of the fragments without the addition of specific primers (as is usual with PCR) to reconstitute the whole-length gene. The generated fragments are suitable for PCR amplification which is performed in order to recombine the fragments, yielding a shuffled pool of recombined polynucleotides. The fragments can anneal with each other thus leading to cross-over reactions, since a DNA fragment derived from one template primes DNA synthesis by binding on the homologous position of a related but different template. The products are subsequently cloned as a cleaved unit length molecule into an appropriate expression vector and screened for desired properties in an appropriate host organism. Further variants of DNA shuffling which all use PCR as a central feature for recombining DNA molecules have also been described with random priming (Shao et al., 1998).

The mentioned recombination processes have various limitations. If they are carried out with a population of homologous DNA molecules, having around 99 to ca. 70% sequence homology (see e.g. Joern et al., 2002, where heptamer homology core sequences with variable G+C content are considered essential) recombination will occur. But the desireable option to recombine polynucleotides with lower homology on the DNA level is quite limited. Groups which have studied the bias in recombination sites used during DNA shuffling with DNA segments of low homology, pointed out that this methodology is not very suitable for this task since most of the molecules obtained were parental, i.e. had not undergone recombination, or had been preferentially involved in recombination at sites of highest homology (Schmidt-Dannert et al., 2000; Joern et al., 2002). Mathematical modeling and practical measurement of recombination bias in practice ([Joern et al., 2002]) indicates that regions of higher homology will dominate as preferred recombination positions during DNA shuffling, an effect which was neither desired nor originally discussed or anticipated by the inventors of the mentioned methodology although it can be considered a major antithesis to obtaining the desired randomized recombination. It was noted in particular that where there is little homology between participating molecules there is a disproportionate number of parental molecules which have not undergone any recombination remaining in the population and causing an inordinate increase in the amount (numbers of clones to be screened) of screening necessary to find advantageous recombinants. In fact, erroneously, this methodology has been portrayed as optimal for directed evolution using gene families of low homology (e.g. [Crameri et al., 1998]).

PCR based methods have the further draw-back that it is necessary to clone the resulting recombination products into suitable expression vectors after recombination has occurred. The cloned products can then be tested for their characteristics by, for example, expressing them on a phage in phage display. After the next selection cycle it is again necessary to amplify the polynucleotides in the vector and to clone the resulting shuffled products into a new vector before a new selection round with the recombined molecules can be performed, this, in toto, being quite time and labour consuming, especially if several rounds of recombination and selection are performed. Furthermore, erroneously recombined or incompletely extended molecules occuring during the shuffling process thus decreasing the efficiency of making the library and hence decreasing the quality of the library, since a high quantity of rejects, i.e. non-functional or unusable artefacts are created. Known recombination methods naturally aim at achieving high quantities of recombination products. However, the output in terms of physiologically active recombinants is lower than expected. It is therefore presently a goal of various research projects to further increase the number of variants. WO 01/75091 shows a combination process for CDR-regions of antibody fragments. The basic idea is to amplify the CDR regions of one molecule and to transfer this CDR region into a so called master framework. Thus it is possible to transfer any CDR into a selected framework resulting in composite, recombined antibody molecules. Thus, a variability in the antibody binding site is created since also CDRs which usually would not create a binding region together can be brought together thereby allowing new folding structures. Therefore a random recombination of CDR regions may be achieved. The created variants are captured in large libraries and analysed for their characteristics. The drawback of this technology is the fairly time consuming process of creation of the library by PCR and especially the performing of further recombination rounds since also with this method it is necessary to repeat the process of PCR-amplification of the CDRs and cloning in the right positions of the master framework after each selection process. Thus the method is not efficient enough to allow directed recombination.

Furthermore, in developing an efficient recombination method it is necessary that important structural motifs which may have been selected for or which have accumulated during selection rounds are not disrupted. This problem is known in the state of the art (Joern et al., 2002). The so called "schema disruption" describes the extent to which a cross-over disrupts beneficial sequences analogous to its use in computer science and optimisation of genetic algorithms. Also nature shows this principal in order to increase the rate of evolution by allowing higher recombination frequencies in between genomic regions encoding functional or structural domains (exons), i.e. recombination taking place within the long intron encoding regions. This hypothesis is supported by recent data (Voigt et al., 2002).

As a result recombination methods are known in the state of the art, wherein exons are shuffled to mimic natural processes in order to evolve proteins by generating and screening libraries of exon-shuffled genes. This idea was initially conceived as a principle for library construction (Fisch et al., 1996 ) utilising a self-splicing intron in Escherichia coli, for peptide phage-display libraries, allowing site-specific cre/loxP recombination within the intron. This system has, however, not found general acceptance and appears to suffer from genetic instability. Further in vitro methods are known (Kolkman and Stemmer, 2001). Exons or combinations of exons encoding domains are amplified using mixtures of chimeric oligonucleotides which determine which exons are spliced together. Mixtures of PCR fragments are then combinatorially assembled into full-length genes using a self-priming overlap polymerase reaction (Crameri et al., 1996). Recombination occurs when an exon from one gene is connected to an exon from a different gene. The drawback of this method, as described above, results from the central reliance on homologous regions. Furthermore these systems are confined to cloning and expression in eukaryotic cells.

It has also been proposed to recombine exons by the use of restriction enzyme sites present in the intron regions or at non-essentail sites between polypeptide coding regions (EP 1 149 905 A1). Preferably enzymes are used which generate non-palindromic sticky ends to allow an ordered reassembly in order to prevent the formation of head-to-head and tail-to-tail ligated molecules. The restricted molecules are ligated thus allowing the formation of recombined exons. Still recombination can only be performed between homologue sequences. If unrelated subsets are recombined non-functional Genes will be created.

It is an object of the invention to provide an efficient recombination method with a higher yield of physiologically useful variants.

The problem is solved by the independent claims. Advantageous embodiments are defined in the dependent claims.

The present invention allows an ordered recombination also between different groups or regions of molecules.

in theory it is possible to shuffle DNA between any clones. However if the resulting shuffled gene is to be functional with respect to expression and activity, the clones to be shuffled have to be related or even identical with the exception of a low level of mutations otherwise leading to non-functional genes, since otherwise expression products would bee generated that will not fold into a functional structure. Often sections of a molecule will freely recombine while still retaining function. However, there are critical sections wherein it is important to preserve a given structure ("schema") which may vary widely within the population of molecules to be recombined. In the state of the art it is, therefore, either necessary to avoid recombination between such polynucleotides by physically/spatially separating the different groups or to accept that high numbers of non-functional recombinants are created between the subgroups present in the population.

According to a first embodiment of the present invention a recombination process for recombining a population of heterologous polynucleotides comprising variant groups and/or regions is provided, wherein recombination is performed within one group of polynucleotides and/or between defined regions of polynucleotides by using different restriction sites in different groups and/or regions of polynucleotides.

For the purposes of this invention the expression Polynucleotide comprises any nucleotide sequence of natural or synthetic origin which can also comprise analogous chemical structures. Heterologous means a variation in at least one nucleotide.

The recombination method is based on the use of restriction enzyme cleavage to generate useful ordered diversity in the recombination products. The invention allows to recombine different groups of polynucleotides or different regions of polynucleotides in one reaction thereby ensuring that recombination can take place only within one group of polynucleotides or between defined regions of the polynucleotides. Thus the overall structure of the polynucleotides is preserved preventing the generation of false recombinants between different groups or between different regions of the polynucleotide which would enlarge the amount of rejects or dysfunctional structures (disrupted schema) in the library. As a result it is possible to carry out the process of the present invention, in one reaction, with mixtures of several distantly related or unrelated groups (subsets) of polynucleotides, which recombine due to the use of different restriction sites independently, thus increasing diversity while maintaining structurally conserved regions. The method can be carried out without separation of individual components or clones. The method allows the generation of highly diverse polynucleotide populations and nevertheless ensures with a higher probability of novel recombinant molecules maintaining useful structure, as for example, their correct folding into secondary and tertiary structures. This may be at the level of the polynucleotide or in certain embodiments properties of a selected expressed peptide or protein encoded by the recombinant polynucleotide. This teaching thus allows the parallel recombination of different groups of polynucleotides or between different regions of the polynucleotide in one ordered reaction. Also this feature allows to carry out recombination between certain structural units or subunits of the different groups in some regions and to avoid the same in other, critical regions, which for example could lead to a useless fold in the expression product. This feature greatly enhances diversity and still leads to an extremely high number of correctly folded proteins. This characteristic will be discussed in more detail with a preferred embodiment of the invention.

According to a further aspect of the present invention a recombination process for recombining a population of variant polynucleotides is provided, - wherein the polynucleotides comprise at least one section defining a structural unit and at least one section defining an interstructural motif; - wherein at least two variant interstructural motifs are present in the population; - wherein variant interstructural motifs are recombined separately by using different restriction sites in different interstructural motifs.

This embodiment ensures the preservation of the overall structure of the polynucleotides which are recombined thus allowing to recombine polynucleotides with variant interstructural motifs wherein the overall structural frame is preserved. The variants can comprise for example different interstructural motifs defining different regions being present between different structural units and/or interstructural motifs being present in the polynucleotide which vary in the population of polynucleotides thus defining different groups of polynucleotides with variant interstructural motifs. This can be the case if different subclasses of a molecule exist, meaning, for example, that the expression products have a slightly different folding structure at least in some sections of the molecule. The interstructural motifs can encode a polypeptide region which makes up the scaffold region of a protein as for example in an antibody, in enzymes, receptors and the like. The scaffold regions ensure that the regions making up the specific characteristics of the molecule are held in the correct position thus allowing e.g. target binding or catalytic activity. Preferred examples are the different subclasses of an antibody. It is physiologically important to preserve the framework of an antibody or antibody fragment in order to preserve the orientation and in order to ensure that the molecules fold correctly. Otherwise many recombinants will occur that have a wrong overall folding structure and thus cannot depict the desired characteristics even though the recombined structural units or structural subunits would have been advantageous if presented in the correct fold. The invention overcomes this problem by using different restriction sites for each variant interstructural motif that participates in recombination and which is not allowed to recombine with different interstructural motifs thus assuring that a unique restriction site is present only in one particular subset.

Furthermore this embodiment allows the recombination of structural units as a whole. Structural units are for example polynucleotide sections encoding proteins, protein subunits, protein domains, polypeptide fragments or polypeptide secondary or supersecondary structures. Further a structural unit can comprise a polynucleotide section with special characteristics that are created by the polynucleotide itself. Recombination takes place at least at a restriction site present in the so called interstructural motifs. The interstructural motif can comprise a polynucleotide section encoding for example a scaffold region of a polypeptide or a linker and can thus be a structural domain itself. The interstructural motif can also comprise part of a vector sequence, a non-coding region or any introduced polynucleotide. By use of restriction sites that are present at the interstructural motifs the structural units can be shuffled as a whole. Thus the disruption of structural schemata's as described above in the state of the art is avoided since beneficial sequences ("schema") are preserved during recombination mimicking natural evolution which in higher organisms (eukaryotes) is significantly accelerated or can be considered to rely to a large extent on the shuffling of exons.

Therefore it is advantageous to control the position and amount of restriction sites within the polynucleotides that are recombined especially since the invention allows the ordered recombination of molecules that are not highly related on the nucleotide level but share homology on the primary, secondary or tertiary polypeptide level, since by introduction of a restriction site at an appropriate position in the polynucleotide a recombination site is created allowing the recombination of molecules that are homologous only at the polypeptide level.

With the present invention it is thus possible to evolve molecules which are made of structural units, as for example different protein domains by recombination processes. The method is very efficient in order to evolve molecules from for example variant gene families. Many, especially eukaryotic proteins depict a modular structure wherein the modules (usually domains) fold independently and can therefore be shuffled without destroying the fold of that module. Examples of such "mobile" domains which can also be shuffled between different proteins are disclosed in Kolkmann and Stemmer, 2001, which is incorporated herewith by reference. Thus it is possible to create variants or even new proteins by shuffling modules. Further examples of such modular molecules are e.g. antibodies, enzymes as e.g. TIM, triose-phosphat isomerase, enzyme inhibitors, receptors as T-cell receptors and the like. For example the TIM is made of an ordered framework structure consisting of barrels of ß-strands and α helices and loops for the substrate binding. With the teaching of the present invention one can preserve the scaffold structure by inserting or choosing restriction sites in the interstructural motif encoding the scaffold regions of the polypeptide. However, the method is not limited to natural populations of diverse genes even though this might be advantageous in some cases (see below). The basic principals of the invention are also suitable for the recombination and evolution of hypothetical genes wherein structural units or subunits are being evolved. With the methods of the present invention it is possible to maintain the "schema" or structural scaffold within functionally related sets. Basically all molecules which are made of structural units and/or structural subunits, wherein an ordered structure needs to be maintained can be recombined within the teachings of the invention preserving the overall structure and thus the scaffold of the molecules if necessary. For the purpose of this invention the terms "structural unit or subunit" or "schema" comprise any selected polynucleotide region with any functional property on any molecular level as e.g. primary, secondary, tertiary or superstructure or domain or interdomain-properties, including the definition of Joern et al., 2002 which is fully incorporated by reference or even hypothetical or empirical selections. The actual content of the broad definition of "structural unit or subunit" and "schema" for the present invention will depend on the knowledge available to the person skiled in the art when applying the teaching of the invention, with respect to what motifs, structural elements or selected subgroups are functionally relevant. Even without this detailed knowledge related subgroups may be arbitrarily (empirically) defined as apparently relevant, as is the case for the related framework subgroups seen amongst immunoglobulin variable regions.

Preferably at least one restriction enzyme is used which creates a non-palindromic cohesive end. The usage of non-palindromic cohesive ends ensures that the cleaved fragments will reassemble during ligation in an ordered way thus avoiding the formation of head-to-head or tail-to-tail ligated products which would decrease recombination efficiency. Because of this feature the efficiency of the library is greatly enhanced since less rejects are produced.

These aspects concerning the shuffling of structural units and the use of restriction endonucleases which create non-palindromic cohesive ends are also important for another embodiment of the invention which provides a recombination process for recombining at least two polynucleotides each encoding a polypeptide comprising at least a structural unit and at least one interstructural motif, which comprises
- cleaving the polynucleotides with at least one restriction enzyme creating a non-palindromic cohesive end thus creating a recombination site;
- wherein a restriction site is present in the interstructural motif which comprises a coding region of the polynucleotide;
- ligating the resulting mixture of fragments.

This embodiment is advantageous due to the above explained shuffling of structural units. Further this embodiment allows the recombination of polynucleotides within the coding regions of a polynucleotide. Thus the recombinant polynucleotides can be expressed in prokaryotic cells since no introns or exons are present which would make the expression in eukaryotes necessary.

Preferably a PRE is used as a restriction enzyme in order to create non-palindromic cohesive ends. *Polynucleotide releasing enzymes* (PREs) comprise proteins possessing endonucleolytic activity, where the cleavage of a polynucleotide polymer takes place at a site physically removed, but at a fixed distance, measured as a specific number of nucleotide bases, from the specific recognition or binding site (lists of such enzymes and their properties can be accessed at http://rebase.neb.com). The first type of enzyme recognised as having such properties were the type IIs restriction endonucleases ([Podhajska and Szybalski, 1985 ];[Szybalski, 1985]). The term is now taken to encompass also chimerical protein genes encoding products which contain the unspecific cleavage domain of the endonuclease joined to a novel specific DNA binding site [Aggarwal and Wah, 1998].

It will be appreciated by a person skilled in the art that in case different restriction sites are used in order to allow an ordered recombination between different regions or groups of polynucleotides as explained above it is sufficient to create unique restriction sites. The restriction site comprises the position where the actual cleavage takes place and preferably a cohesive end is created. The restriction site can be distinct from the recognition (binding) site in case a PRE is used. This can either be achieved by the use of different restriction enzymes which recognise different recognition (binding) sites and which create different cohesive ends by cleavage. Thus for example a different enzyme is used for each subclass of a molecule in case recombination between subclasses is expected to cause problems. However, this aim may also be achieved by the use of the same restriction endonuclease if it is ensured that the nucleotides at the actual cleavage site vary between the different groups of polypeptides or regions and are unique for each group or region which should recombine with each other. For this embodiment it is advantageous to use a PRE. The recognition site would be the same for each variant group or region but the nucleotides at the adjacent cleavage site would vary between the different groups or regions due to nucleotide sequence variations thus preventing recombination between different groups or regions. The uniqueness of the resulting recombination site (cohesive end) for each group or region can for example be ensured by site specific mutagenesis at the actual cleavage site.

It is preferred that the polynucleotides are present in a vector during recombination. Of course per definition also the polynucleotide itself can be a vector construct. The recombination of whole vector constructs has the advantage that cloning steps after recombination are avoided and after the recombination process of the present invention the polynucleotides are again present in the vector and can be directly used for selection avoiding time and labour consuming intermediate cloning steps.

In case a circular polynucleotide as e.g. a vector is recombined at least two restriction enzymes should be used. Preferably the cleaved circular polynucleotides ligate and recombine at high concentrations allowing the formation of concatemers. Suitable concentrations are namely >50µg/ml up to 2mg/ml. Preferentially a concentration of more than 200µg/ml is used. The concatemers are resolved by cleavage with a restriction enzyme releasing recombined vectors which are recirculised by ligation at low DNA concentrations (<50µg/ml) and are ready for use. Preferably the first restriction enzyme is a PRE enzyme which creates non-palindromic cohesive ends ensuring that upon ligation the original structure of the polynucleotides is re-established in the recombined molecule preventing head-to-head and tail-to-tail ligated recombined molecules. Since the circular polynucleotides which are linearised by the use of the first PRE enzyme create ligation constructs comprising several ligated vector sequences ligated into a concatemer. it is necessary to then resolve these concatemers with a second enzyme which releases single recombined polynucleotides allowing the recircularisation of the polynucleotide by ligation. This resolving restriction enzyme can optionally be a PRE. Preferably the restriction sites for recombination are present in the interstructural motif.

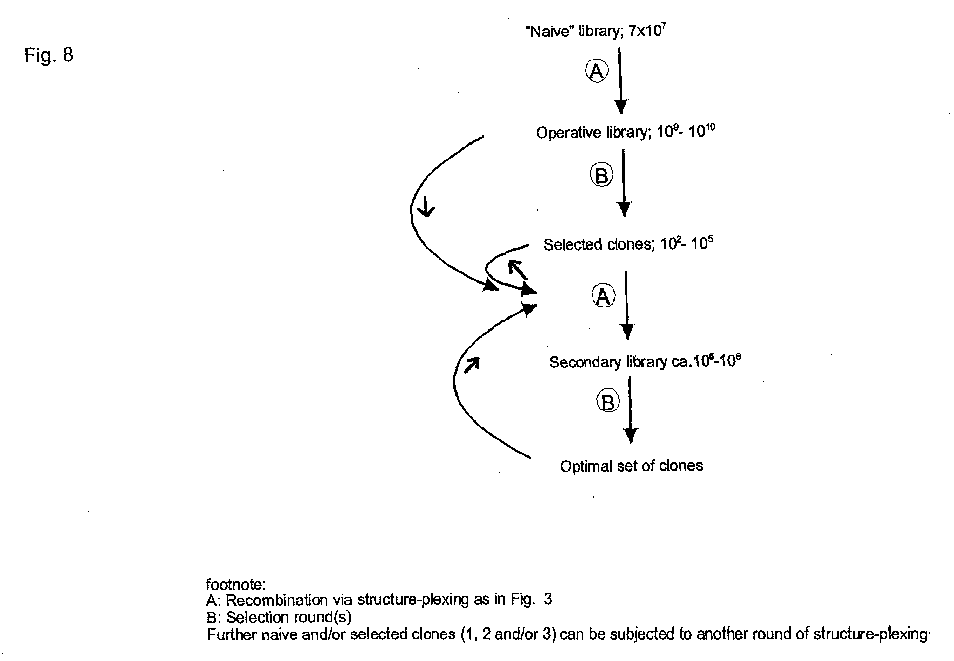

In a preferred embodiment, where after cleavage with a first PRE specific for a defined subgroup, the molecules are ligated to form a concatemer, it is also revealed in the invention that resolution at a second restriction site within the subgroup-specific structure, followed by circularization of the vector will also lead to subgroup-specific recombination at the second restriction site, whether or not this second restriction site is subgroup specific or not (as shown in Fig.8). The latter point relies on the fact that group specific concatemers have been formed and that ring closure will be formed due to low DNA concentration.

According to the invention it is possible to choose appropriate binding sites for one or more restriction endonucleases which are already present in the polynucleotide or to introduce such sites by site specific mutation at predefined positions which are appropriate for recombination. Preferentially the binding sites for one or more restriction enzymes are introduced at defined positions (interstructural motifs) between gene segments encoding heterologous structural units or subunits as explained above. For this purpose it is also possible to introduce interstructural motifs between structural units as subunits in order to create appropriate locations. This introduced interstructural motif can be any nucleotide sequence and length and serves as an equivalent to an intron by recombination without destruction of the structural units. Of course also an intron may be introduced as an interstructural motif. Preferably the intron is removed after recombination, for example by using a self-splicing intron. However, the added interstructural motif may remain in the created polynucleotide if the frame of the encoded polypeptide is not destroyed.

Further a method is provided for evolving a molecule by selection and recombination which comprises the use of a recombination process as outlined above. In detail the method comprises:
a.) creating a starting library by cloning the polynucleotides to be recombined into an expression vector if the polynucleotide itself is not a vector
b.) recombining the library members with the process according to the invention as described above
c.) selecting candidates with improved characteristics
d.) optionally performing further rounds of recombination and selection.

Optionally the polynucleotides are mutagenised before or after selection in order to create additional diversity. It is also possible to mutagenise the polynucleotides before the starting library is created. It is even possible to start the population from one parental polynucleotide which is mutagenised in order to create variants. Conventional mutagenesis methods such as for example error-prone PCR, cassette mutagenesis and chemical mutagenesis methods may be referred to.

Preferably the polynucleotides are expressed such that it is possible to link the genotype to the phenotype. The library can be, for example, an expression library wherein the polynucleotides are expressed in a host cell. The recombined polynucleotides are expressed in a cell and selection is performed by screening or selecting for a particular phenotype among the cell clones as for example whether they can deliver a certain resistance to the host cells. The expression products also be screened for a certain catalytic activity or the like.

Various expression systems are known and applicable, including systems comprising: prokaryotes (e.g. E.coli) transformed with, e.g., recombinant phage, phagemid, plasmid or cosmid expression vectors; eukaryotic systems as for example yeast transformed with, for example, yeast expression vectors; insect cell systems transformed with, for example, viral expression vectors; plant cell systems transfected with, for example, viral or bacterial expression vectors; animal cell systems transfected with, for example, adenovirus expression vectors.

The expression vectors may include a prokaryotic replicon, an appropriate promoter capable of directing expression of the genes in the respective host cell. Typical prokaryotic vector plasmids are: pUC18, pUC19, pBR322 and pBR329 available from Biorad laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA). A typical mammalian cell vector is pSVL available from Pharmacia (Piscataway, NJ, USA). Useful yeast plasmid vectors are for example pRS403-406 and pRS413-416 and are available from Stratagene Cloning Systems (La Jolla, CA 92037, USA). Plasmids pRS403, pRS404, pRS405 and pRS406 are Yeast Integrating plasmids (Yips) and incorporate yeast selectable markers. Plasmids pRS413-416 are Yeast Centromere plasmids (Ycps).

In one embodiment the selection procedure comprises the selection for affinity to a defined target. In order to evolve molecules with certain binding characteristics it is preferred to create a display library. The molecules that are displayed on the surface of the host can be easily screened for their binding characteristics by contacting them to the target molecule which is for example immobilised on a carrier. A phage or phagemid library is preferred because of the technical advantages of this vector system as to be outlined below.

The display of proteins or polypeptides on the surface of bacteriophages fused to one of the phage coat proteins as for example pill or pVIII, provides a powerful tool for the selection of specific ligands. First a polynucleotide encoding a protein or polypeptide for display is cloned into a phage, wherein the cloned polynucleotide is expressed fused to the coat-anchoring part of one of the phage coat proteins (pIII or pVIII in case of a filamentous phage) such that the foreign protein or polypeptide is displayed on the surface of the phage. The phage displaying the protein or polypeptide with the desired properties is then selected (e.g. by affinity methods) thereby providing a genotype (linked to a phenotype) that can be sequenced, multiplied and transferred to other expression systems.

Alternatively, the foreign protein or polypetide may be expressed using a phagemid vector that can be packaged as a single stranded nucleic acid in a bacteriophage coat. When phagemid vectors are employed a "helper phage" is used which supplies the functions of replication and packaging of the phagemid nucleic acid.

Several methods of selecting phage expressing a protein or peptide with a desired specificity are known in the state of the art and applicable. A widely used method is the so called panning, in which phage stocks displaying ligands are exposed to solid phase coupled target molecules, e.g. using affinity selection. Any method for selecting phages may be used with the present invention.

The use of phage display to isolate ligands that bind biologically relevant molecules is known (Katz (1997) Annual Rev. Biophy. Biomol. Struct. 26, 27-45and Hoogenboom et al. (1998) Immunotechnology 4(1), 1-20). Also proteins and multimeric proteins have been successfully phage displayed (EP 0349578A; EP 0527839A; EP 0589877A; Chiswell and McCafferty, 1992, Trends Biotechnol. 10, 80-84). In addition functional antibody fragments have been expressed (see above). Further information on the principles and practice of phage display is provided in *Phage display of peptides and proteins: a laboratory manual* by Ed Kay, Winter and McCafferty (1996) Academic Press, Inc ISBN 0-12-402380-0, the disclosure of which is incorporated herein fully by reference.

The generated evolved polypeptide sequences are expressed in a host cell and can be recovered and purified from recombinant cell cultures by well-known techniques including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Also high performance liquid chromatography (HPLC) can be employed for purification.

It will be appreciated by a person skilled in the art that the methods of the present invention can be used to produce recombinant polynucleotides encoding antibodies or antibody fragments especially polypeptides encoding a variable domain of an antibody. According to a special embodiment of the invention thus an advantageous process is provided for recombining antibodies and/or antibody fragments. The variable domain may be for example an IgG, IgM, IgA, IgD or IgE variable domain.

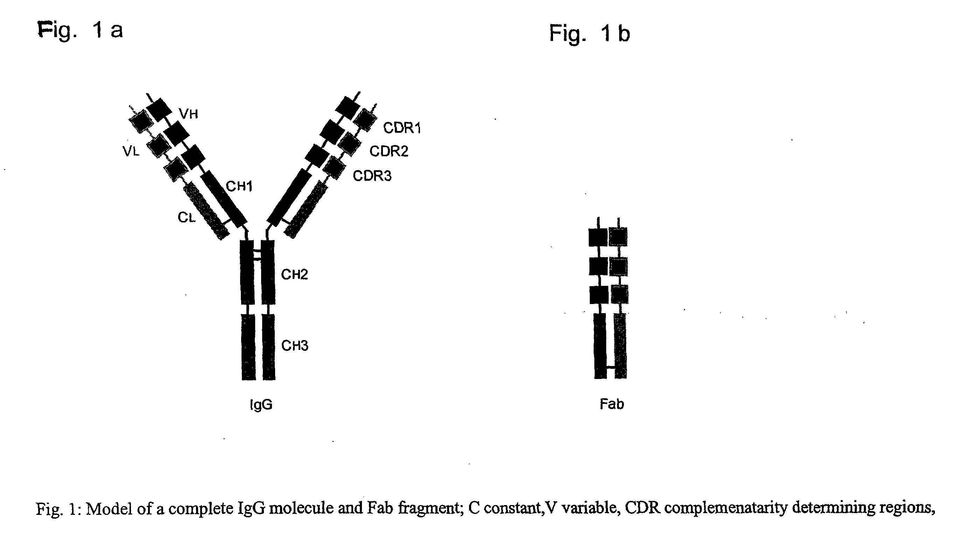

Structurally the simplest antibody (IgG) comprises four polypeptides, two heavy (H) chains and two light (L) chains which are connected by disulphide bonds as it is shown in Fig.1. The light chains exist in two distinct forms, called kappa (K) and (λ). Each chain has a constant region (C) and a variable region (V). Each chain is made up of a series of domains. The light chains have two domains, one corresponding to the C region and one to the V region. The heavy chains have four domains, one corresponding to the V region and three domains in the C region. The antibody has two arms wherein each arm is a Fab region and comprises a binding site for an antigen. Each Fab (see Fig. 1a) region has a VL and a VH region associated with each other. It is this pair of V regions that differ from one antibody to another (owing to amino acid variations). Each V region is made up from three complementarity determining regions (CDR) separated by four framework regions (FR). The CDRs are the most variable part of the variable region and they perform the critical antigen binding function. The FRs ensure that the CDRs are held in the correct place in order to allow binding. Thus the correct fold of the framework is important, moreover it has been shown that single framework amino-acids can take part in antigen binding. The constant regions CH1 and CL stabilise the heterodimer by noncovalent association. This allows more variability in the VH and VL domains, since they are not limited by stabilising needs. Different subclasses of variable domains exist that are different in dimension and fold. Three distinct V _{H} "clans", referred to as antibody-subclasses (heavy-chain variable subclasses), were defined by Kirkham et al., (1992), based on protein structural conservation in two (FR1 and FR3) of the three framework intervals, which are essentially the same as the three "Kabat" groups previously defined on the basis of the DNA sequences which are described in Kabat et al. (1991).

It has been shown that the function of binding agents can be performed by fragments of a whole antibody. Examples of binding fragments are e.g. the Fab fragment consisting of the VL, VH, CL and CH1 domains, the Fd fragment consisting of the VL and VH domains, the Fv fragment consisting of the VL and VH domains of a single arm of an antibody the dAB fragment which consists of a VH fragment, isolated CDR regions and F(ab')₂ fragments, a bivalent fragment comprising two Fab fragments linked by a disulphide bridge at the hinge region. Since the Fv fragment lacks the stabilising constant regions they can be linked by for example a synthetic linker in order to ensure the creation of an antigen binding site (ABS).

All these different antibody fragments can be recombined according to the present invention, even though the recombination of Fab fragments is preferred due to stability reasons.

According to a further aspect of the invention it is possible to recombine polynucleotides encoding structural units which are made up of structural subunits being connected by interstructural motifs. One important example of this embodiment comprises variable fragments of an antibody. In this embodiment the variable fragment is the structural unit which is made of CDRs as structural subunits that are connected by framework regions as interstructural motifs. According to the present invention either the structural units as a whole and/ or the structural subunits can be recombined thus increasing diversity. Also the structural subunits can comprise protein domains, protein fragments, protein secondary or supersecondary structures and the like. The shuffling of whole subunits has the same advantage as explained above for the shuffling of structural units.

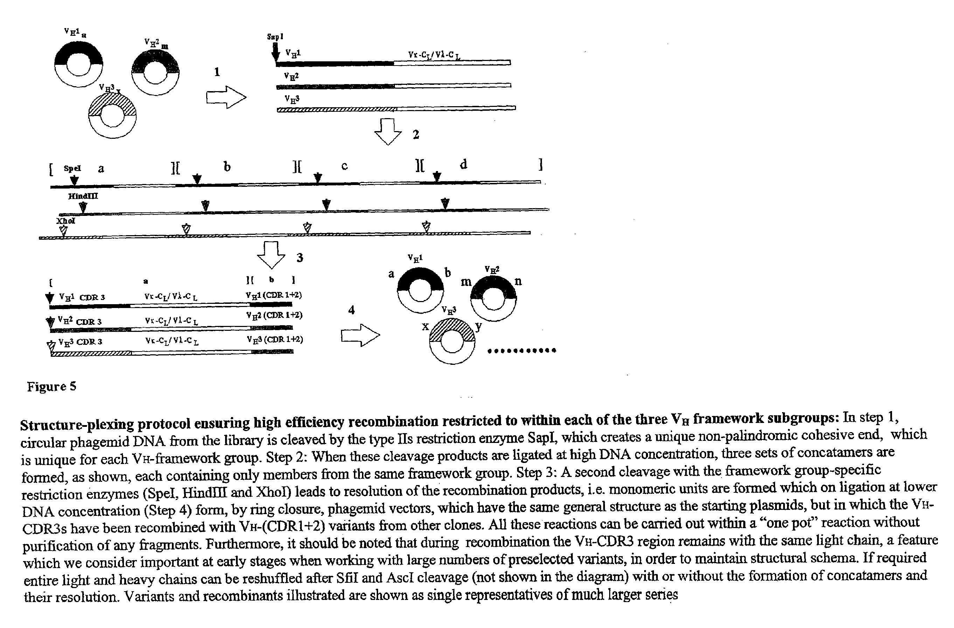

Thus a recombination process for recombining a population of polynucleotides with variant interstructural motifs is provided wherein the polynucleotides comprise a section encoding at least a variable fragment of an antibody as a structural unit, wherein the CDR regions of the variable fragment define the structural subunits that are recombined with each other and at least the FRs define the interstructural motifs, wherein at least two different subclasses of variable fragments are encoded by the polynucleotide population, wherein the framework of the different subclasses is preserved during recombination by allowing independent recombination of the different groups of subclasses (see Figure 5).

According to one embodiment FR3 is used for recombination. The different subclasses thus comprise a different restriction site in the polynucleotide section encoding the FR3 thus allowing shuffling of the region. This embodiment is advantageous since the CDR-3 is the most variable CDR.

According to a preferred embodiment the restriction site preceedes the heavy-chain coding sequence thus allowing shuffling of V_{H}CDR1+2, and keeping the V_{H}-CDR3 region together with the same light chain. This embodiment is preferred since the CDR3-light chain combination is considered as a single "schema" which should not be disrupted during initial selection where affinities are expected to be relatively suboptimal. In this embodiment in a later step, light and heavy chains from the selected population would be shuffled to obtain further independent diversity.

According to one embodiment the polynucleotides are present in a display vector. The vector can comprise at least the variable fragments of either the light or the heavy chain whereby two different libraries are created, each containing several subclasses. Recombination of CDR regions is allowed within each subclass of each library. The vectors comprising the variable fragments of the light chain and the vectors comprising the variable fragments of the heavy chain can therefore be recombined separately in vitro according to the process of the present invention and can be brought together in one host cell in order to allow the display of a functional binding antibody fragment.

Preferably the vectors comprise at least the variable fragments of the light and the heavy chain. This embodiments allows recombination between the CDRs of either the light or the heavy chain as described above and also the recombination of the heavy fragments against the light fragments by using restriction sites for recombination which are present between the fragments to be recombined. In case the fragments are Fab fragments the additional restriction site for recombination can be present in a region which is part of the vector. In case Fv regions are shuffled at least one recombination site for shuffling light against heavy variable fragments are preferably present in the section encoding a linker region connecting the variable heavy fragment to the variable light fragment. The additional recombination between the light and the heavy fragments increases diversity and also ensures that the host cells are transformed with a construct that encodes the variable heavy and the variable light fragment thus ensuring the display of a functional binding fragment. By using different restriction sites it can be ensured that the shuffling of the light against the heavy fragment is group specific, for example is only allowed between the same subclass.

The polynucleotides comprising the antibody fragments can be generated by producing a library of antibody genes which includes a set of variable regions encoding a large diverse and random group of specificities derived from animal or human immunoglobins by amplification or cloning diverse genomic fragments or cDNAs of antibody mRNAs found in antibody producing tissue. Genomic DNA encoding antibodies or cDNAs of antibody mRNA can be obtained from any antibody-producing cells, such as spleen cells, peripheral blood cells, lymph nodes, inflammatory tissue cells and bone marrow cells or can be obtained from a genomic library or cDNA library of B cells. The antibody-producing cells can be of human or non-human origin; genomic DNA or mRNA can be obtained directly from tissue or can be obtained after a treatment of the tissue in order remove cells or to increase the concentration of antibody-producing cells. Also the antibody-producing cells can be stimulated by an agent which stimulates the antibody mRNA production as for example lipopolysaccharides, before the DNA is obtained.

The antibody encoding DNA or mRNA (respective cDNA) can be amplified and cloned using standard techniques such as PCR using appropriately selected primers in order to produce sufficient quantities of the DNA and to modify the DNA in such a manner that it can be introduced as an insert in the chosen expression vector and in order to introduce the desired restriction sites for recombination and possibly resolution. As soon as the fragments are present in an expression vector they can serve as starting library.

It is preferred to create the starting library from cDNA of immunised donors. Special reference to the cDNA library used in Stryhn et al., 1996 and Andersen et al., 1996 is made as the starting point for construction of the initial library which disclosures are incorporated herein fully by reference. The cDNA library according to the methods described in these documents was created with extreme care in order to prepare a large number of individual clones wherein it is clear to a person skilled in the art that a high class starting library can be created from such a high class cDNA material with respect to quality and diversity.

According to another embodiment of the present invention for creation of a naive human antibody library - whereby naive refers to the fact that none of the individuals from whom immunoglobulin mRNA was obtained had been challenged with a particular antigen - immunoglobulin gene fragments were generated from cDNA which was generated from a large number of individuals with a wide range of histocompatibility alleles (MHC/HLA groups). With this large library it is expected that several different antibodies can be obtained against any particular target, some of which will derive entirely from an individual with a particular HLA genotype. Such an antibody would then be particularly suitable for clinical applications in individuals with that particular HLA genotype. Thus the method of the present invention comprises potential advantages in the area of semi-personalized medicine and also provides the possibility of so called "ethnologically-oriented personal medicine", e.g. where the initial human antibody library is generated from cDNA is derived from individuals of particular ethnological groups, as for example African, Hispanic or Japanese people.

Another advantageous aspect of the preferred embodiment lies in the iterative recombination at specific sites between structural domains, in the case of the framework regions lying between the CDR loops which influence the spacing of the loops. These can vary according to the respective Kabat subclass (for example in size). According to the preferred embodiment recombination only takes place within the framework regions of one subclass thus preserving the framework and fold of the recombined molecule as described above. This aspect is a powerful way to increase structurally stable diversity both during the construction of the initial library as well as in further steps during the selection and amplification procedure. A higher stability of the obtained antibodies or antibody fragments would also improve the half-time of the circulating antibody or antibody fragment which is advantageous if it is used as a pharmaceutical.

Obtaining antibodies from antibody libraries which are more likely to fold correctly is advantageous over methods which do not consider this aspect, eventually leading to molecules which are not folded correctly. Often this will lead to additional rejects lowering the efficiency of the library, subsequent to a recombination process. In some cases the consequences can be even more severe leading to immune reactions. Correctly folded antibodies have the lowest likelihood of having developed novel antigenic/immunogenic properties, thus recombination within the antibody gene population which is also necessary for obtaining products with high affinity and specificity should preferentially maintain the framework structure. This is an advantage over methods known in the state of the art for creating large antibody libraries as for example random recombination of CDRs or replacement of natural CDRs with synthetic sequences. Known methods do not ensure that the antibodies fold correctly and thus useless or even antigenic/immunogenic antibodies are created which lowers the quality of the library due to the higher number of rejects. Because of this limitation additional recombination or mutagenesis steps which do not preserve the structure should be carefully considered since these mutations can also increase the likelihood of generating harmful antigenic mutations.

It is preferred to use multiple frameworks according to the present invention as opposed to using a single or a few framework regions which remain unaltered during recombination, for the following reasons:
- If only a single framework is used in the library, an individual is unlikely to be able to use more than one product derived from this library, within a period of about ten to twenty years, if an antigenic reaction occurs during the initial treatment with a product from this library. This may be the case if a patient develops an allergic or immune reaction to the product of the library in which epitopes of the framework are recognised. No alternative product from a series of similar antibodies derived from the same library can be used to continue the treatment since the allergic or immune reaction would eventually occur, since the same framework is present. Even after years this patient would be in danger of anaphylaxis if ever treated with a product derived from the same library even if it is designed for a different target or a different disease, since common framework regions will be recognised as allergens. This scenario is prevented by the use of multiple frameworks.
- Many CDRs will be incompatible with the one framework into which they have been inserted, as has been documented for the "grafting of CDRs" from murine to human antibody frameworks in the process of producing "humanised antibodies". By trying to compensate for this disadvantage by increasing the number of sites of recombination, it is only possible to cover an insignificant proportion of the recombinants produced (e.g. if 1000 different clones are isolated after an intermediate selection, and all 6 CDRs are recombined, 10¹⁸ combinations can theoretically be produced, although only ca. 10⁸ to 10⁹ will be created (Carson et al., 2000; Olin et al., 2001).
- If only a single framework is used the useful diversity which has accumulated during somatic maturation of the antibody genes will not be present in the library and may have to be introduced by mutagenesis later - a long and tedious process which may or may not achieve the intended affinity, i.e. it is advantageous to use more than one framework, since the possible diversity that can be generated within the library is dramatically increased.

Comparing the antibody library which can be created according to the present invention to "the state of the art", the advantages are evident. For example Hoogenboom in creating a large non-immune library (from preferentially lgM origin) criticised the low affinity obtained with previous libraries in which synthetic CDRs were used, or where scFv format had been used and stability problems had arisen, and presenting the possibility of having lower antigenicity in a non-immune library as a strong advantage increasing the facility of developing an antibody for clinical use ([Hoogenboom, 1999]. Non-immune libraries are created from individuals that have not been specifically challenged with any particular target. According to a preferred embodiment of the invention an IgG and IgM derived library is created which has the advantage of having greater diversity, which with the inventive method in particular will be increased exponentially during the recombination process. Without prior immunisation it is difficult to generate highly affine antibodies since the frequency of usable H-L pairs is low in a non-immune library. The use of the method according to the invention in a preferred embodiment conserves framework regions, with their CDRs, allowing a huge increase in diversity while keeping the generation of immunogenic epitopes and rejects to a minimum. Furthermore the structural maintenance of the framework subtypes should ensure the creation of novel antibodies in the libraries which can fold correctly, thereby ensuring a higher proportion of selectable antibody structures within the library.

Further evidence for the high quality of a library derived from natural V regions without the use of synthetic CDR rearrangement and substitutions, comes from a comparison of the results obtained with such an unadulterated library ([Sheets et al., 1998]) compared to any other results reported in the literature even without the use of directed evolution techniques. With natural libraries it is possible to achieve much higher affinities than for those obtained with libraries containing synthetic CDRs. CDRs of human origin are further proofread and it is thus unlikely that they are immunogenic. This is an advantage over artificial libraries.

The products achieved by the process of the present invention can be used for deriving human therapeutic antibodies and antibody fragment conjugates.

Antibodies or antibody fragments generated from sequences which are selected from these libraries as described above can be used as diagnostics and/or therapeutic products for the treatment of diseases including immune dysregulation (including psoriasis), cancer, septic and toxic shock, blood haemostasis, nerve regeneration, pain, psychological disorders, appetite dysregulation, sexual dysfunction and wound healing or for vaccines. They can also be used for targeted delivery of other pharmaceuticals to differentiated or neoplastic animal or human cells or as cosmeceuticals.
- Fig. 1a: shows the structure of an antibody
- Fig. 1b: shows the structure of a Fab fragment
- Fig. 2: shows the expression cassette of the huFab vector
- Fig. 3: shows schematically a huFab vector according to another embodiment
- Fig. 4: shows schematically the recombination process according to a preferred embodiment
- Fig. 5: is a schematical view of the recombination of different groups of polynucleotides
- Fig. 6 and 7: show in a cloning diagram how the fragments were generated and cloned into the huFab vector and refers to the example
- Fig. 8: shows in a diagram the method for generating an evolved molecule by selection and recombination
- Fig. 9: shows the forward and back primers used in order to introduce unique restriction sites for each subgroup VH1-VH3 and refers to the example
- Figs. 10a: show examples (Examples 1 and 2) of the recombination
- and 10b: process according to the invention.
- Fig. 11: shows the results of a completely human Fab library used for phage display-based selections on human VEGF (vascular endothelial growth factor) according to the invention (Example 3).
- Fig. 12: shows the results of a completely human Fab library used for phage display-based selections on human IGF (insulin like growth factor) according to the invention (Example 4);
- Fig. 13 a: shows A schematic overview over the selection conducted
- and 13 b: according to examples 3 and 4;
- Figure 14: shows the Phage ELISA as described in example 3;
- Figure 15: shows the Phage ELISA as described in example 4;
- Figure 16: shows the production and purification of soluble Fab fragments;
- Figure 17: shows the kinetic analysis for hVEGF:
- Figure 18: shows the kinetic analysis for hIGF.

It should be noted that variants and recombinants illustrated are shown in the figures as single representatives of much larger series.

The content of Fig.1 has already been explained above.

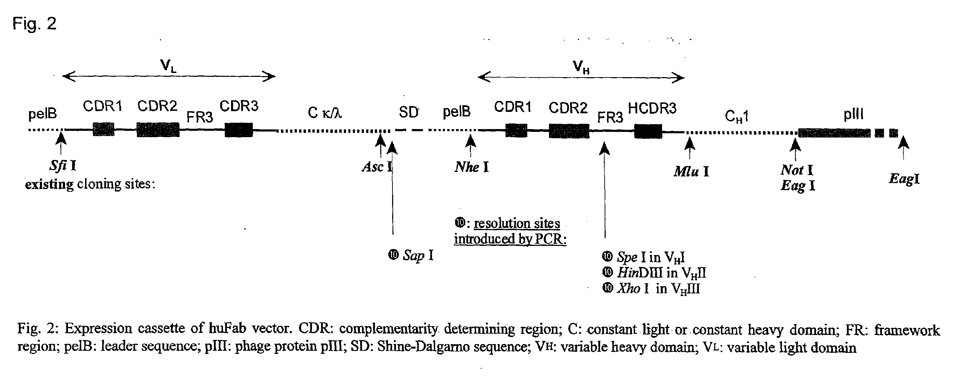

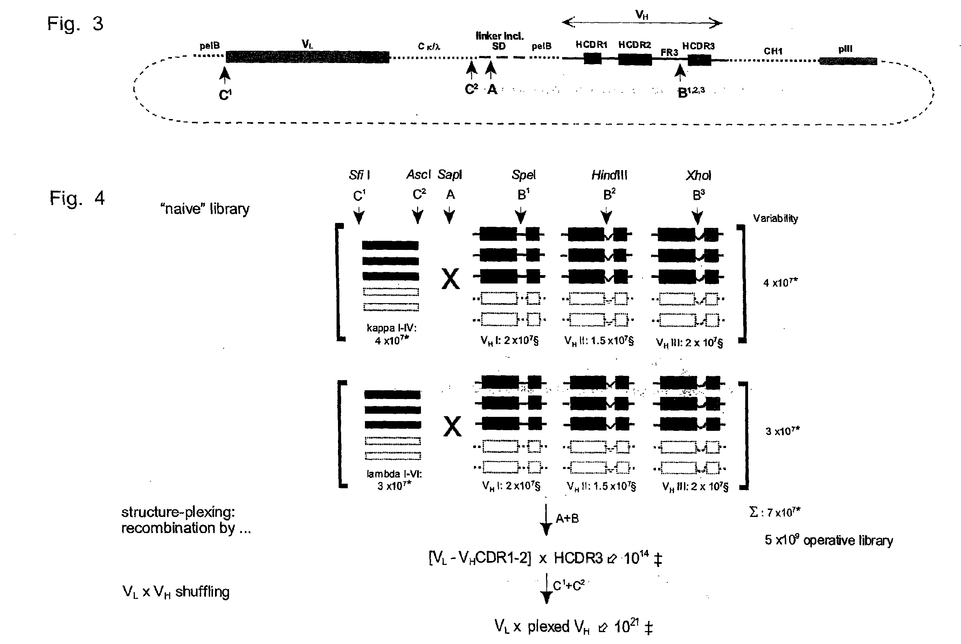

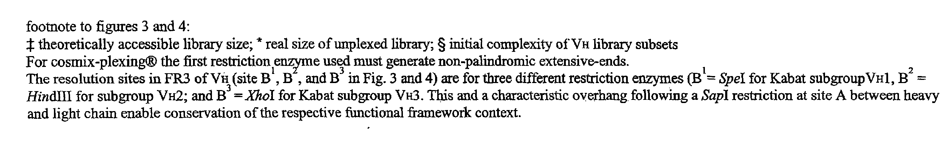

Fig.2 shows the expression cassette of the huFab vector as it is also shown in Fig.3 and Fig.4. The expression cassette is constructed for displaying a Fab fragment. In this embodiment a fragment encoding the variable light fragment of an antibody and a fragment encoding the variable heavy domain of an antibody is cloned in the phagemid display vector. The variable light domain (VL) is cloned 3' of a pelB leader sequence. Also the constant region C is present 3' of the variable light domain (VL) which can be either K or λ. Thus a fragment is expressed which comprises the variable light domain and the constant domain of an antibody. The variable light domain and the constant domain of the light chain are separated from the variable heavy domain (VH) by a vector linker sequence which comprises a Shine-Dalgarno sequence (SD). Adjacent to the linker section a second pelB leader sequence is present. The CDRs1 to 3 are indicated for each fragment as are the framework regions FR3. The constant region CH1 is present 3' of the VH region. PIII is defining the phage protein pill which is the display protein in this embodiment. Also the cloning sites Sfil, Ascl, Mlul, and Notl are indicated which can be used for cloning the fragments into the vector (see example below). Also restriction sites are indicated which can be used for recombination inbetween different subclasses as for example the sites introduced by PCR as Spel in VH1, HindIII in VHII, Xho in VHIII and Sapl. Also the Sfil and the Ascl site can be used for recombination.

Fig.3 shows the Fab-encoding part of the phagemid vector which is used for recombination as explained according to Fig.4.

Fig. 4. demonstrates possible recombinations which can be performed using the indicated restriction sites. According to the preferred embodiment the naïve library is first digested with restriction enzyme A (Sapl) wherein the restriction site is designed such that characteristic overhangs are created following Sapl restriction at site A between heavy and light chain thus allowing recombination only within one subgroup thus enabling conservation of the functional framework context. Sapl creates non-palindromic cohesive ends thus further allowing an ordered recombination between the fragments preventing head-to-head and tail-to-tail formation upon ligation thus increasing the efficiency of the library. Thus three different concatemers are formed, one for each subclass. The concatemers are resolved by the use of the respective B enzyme, wherein B¹ (Spel) resolves the concatemer comprising fragments of subgroup VH1, B² (HindIII) resolves the concatemers comprising the subgroup VH2 and B³ (Xhol) resolves the concatemers of subgroup VH3 and are recircularised by ligation. Thus the VL-V_{H}CDR1-2 fragment is shuffled against the HCDR3 fragment wherein the theoretically accessible library comprises 10¹⁴clones. The diversity can be enhanced by additionally recombining the light fragment against the heavy fragment. This can be performed by using the restriction sites C¹ and C² which refer in this figure to the restriction sites Sfi and Ascl wherein Sfil creates non-palindromic cohesive ends and is used as the first restriction enzyme and wherein Ascl is used as the resolving enzyme for resolving the concatemers. The chains can also be shuffled though without the formation of concatemers.

It is appreciated by a person skilled in the art that it is also possible to shuffle the other CDRs (CDR1 and 2) of the variable heavy domain and also the ones of the variable light domain (CDR1 to 3) by introducing appropriate restriction sites in the respective framework regions. It is also appreciated by a person skilled in the art, that it is possible to create separate libraries for the heavy chain and the light chain fragments. By insertion or using appropriate restriction sites in the framework regions it is possible to shuffle CDRs against each other within the light or heavy fragment library. In order to allow expression of a functional Fab it is necessary according to that embodiment to transform the respective host with a vector of each library or the host itself carries a respective expression cassette in its genome. Thus a variation of the composition in which light and heavy chains are expressed together takes place. Also it is possible to perform in vivo recombination in order to enhance variation. It is also appreciated by a person skilled in the art that instead of three restriction sites for three different enzymes as used in the above described embodiments in order to recombine the CDR3 regions it is also possible to use one restriction enzyme in case it is assured that upon cleavage different cohesive ends are created for each subgroup as it is possible with for example PRE enzymes. Thus it might be necessary to vary the nucleotides at the recombination site which gets created upon cleavage by mutagenesis.

Fig.5 shows how highly efficient recombination is performed within the three different subgroups wherein recombination is restricted to within the three VH framework groups (as explained above). First circular phagemid DNA is cleaved with the typells restriction enzyme Sapl which creates a unique non-palindromic cohesive end which is unique for each framework group wherein the different VH framework groups are indicated by different shadings. As a result of step one the vectors are linearised upon cleavage wherein each subgroup has a unique, group-specific cohesive end. In step 2 the cleavage products are ligated at high DNA concentrations thus forming three sets of concatemers each containing only members of the same framework group since the three different framework groups have different cohesive ends generated on cleavage with Sapl. The brackets indicate the original, linearised vector in the concatemers. In step 3 a second cleavage within the framework group specific restriction enzymes (Spel, HindIII and Xhol) leads to resolution of the recombination products, i.e. monomeric units are formed which on ligation at lower DNA concentrations (Step 4) form by ring closure phagemid vectors, which have the same general structure as the starting plasmids, but in which the VH-CDR3s have been recombined with VH-(CDR1 + 2) variants from other clones. All these reactions can be carried out in a "one pot" reaction without the need for separating or purification of any fragments. According to the shown embodiment it is also possible that during recombination the VH-CDR3 region remains with the same light chain a feature which contributes to maintaining the structural schema and also the stability of the displayed fragments.

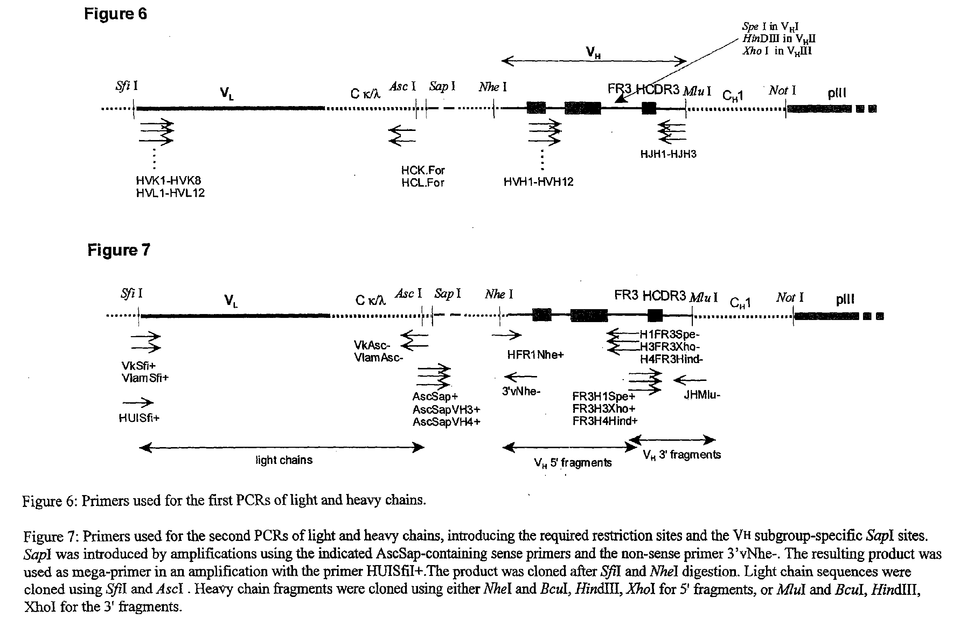

Fig. 6 and 7 show in a cloning diagram how the fragments were generated and cloned into the huFab vector and refers to the example

Fig. 8 demonstrates the selection process. The naive library as described in Fig.4 comprises around 7x10⁷ variants. Those are recombined in step A with each other generating the operative library with about 10⁹ to 10¹⁰ variants. Then in step B at least one selection procedure is performed in order to select candidates with desired properties. The selected clones (10² to 10⁵) are recombined either with themselves and/or other selected clones or with naïve clones (1,2 and/or 3) thus creating a secondary library with about 10⁴-10⁹ variants. Those undergo at least one further selection round in order to obtain the optimal set of clones. It is understood that the process of selection and recombination can be performed as often as necessary in order to obtain the optimal set of clones.

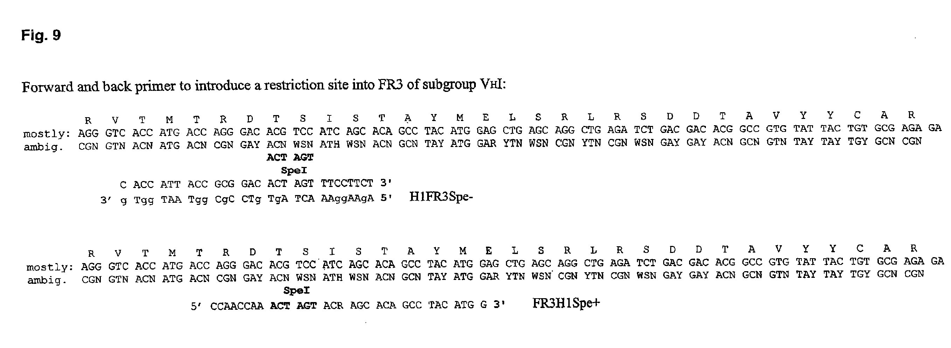

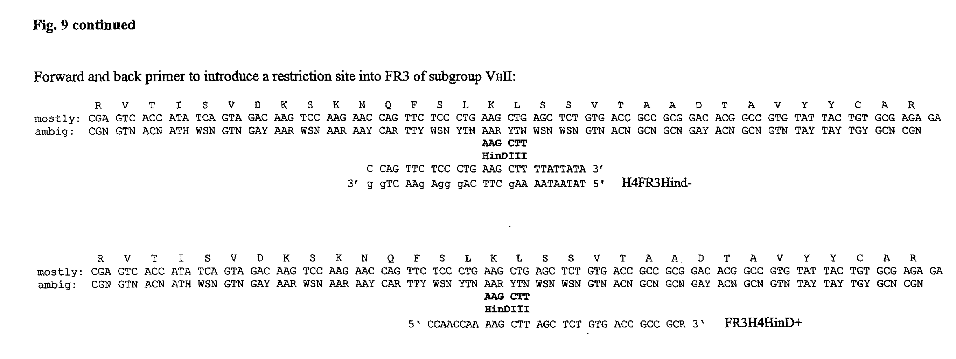

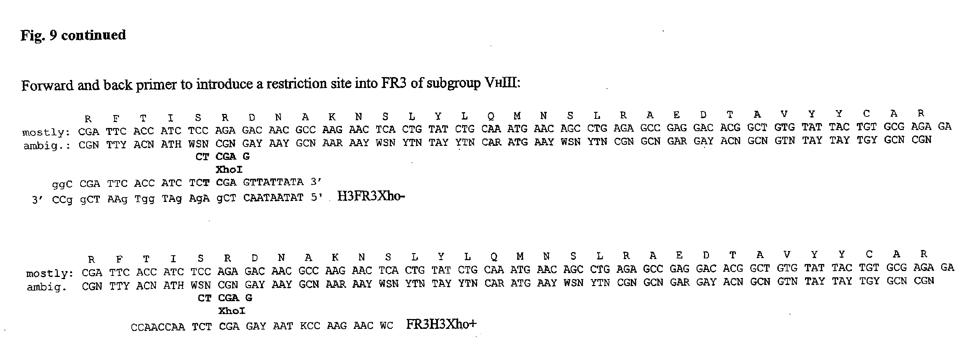

Fig. 9 shows the forward and back primers as described above.

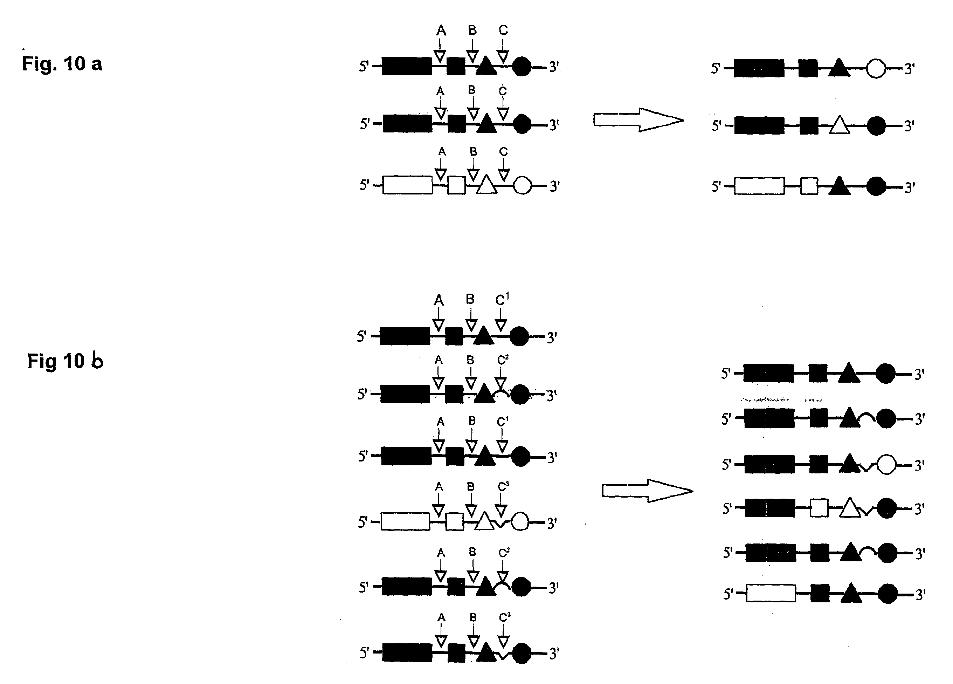

Fig. 10a shows an embodiment of the present invention wherein polynucleotides are recombined that have different interstructural motifs (regions) between the structural units. The structural units encode for example protein domains responsible for the binding of a target. The interstructural motifs encode scaffold regions which ensure the overall correct fold of the polypeptide. In order to allow an ordered recombination different restriction sites 1 to 3 are used for each interstructural motif. These different restriction sites can be achieved by introducing or choosing binding sites for different restriction enzymes or in case a PRE is used the same binding site can be used if it is ensured, that the restriction site varies such that non-palindromic cohesive ends get created that differ from each other preventing recombination between "wrong" interstructural motifs. Due to this process it is ensured that recombination can take place at a high frequency also at regions with low or no homology on the DNA level by introducing appropriate binding sites and further that in the recombined molecules the original structure is rebuilt preventing the generation of unusable rejects with a wrong folding.

Fig.10b shows a different recombination possibility wherein there are different interstructural motifs which can freely recombine and wherein there are three subclasses of interstructural motifs wherein recombination is not supposed to take place between the subclasses. In order to assure the conservation of the overall structure three different restriction sites A to C are used for the three different interstructural motifs comprising different regions. In order to further assure that recombination does not take place between the subclasses (groups) of the third interstructural motif three different restriction sites C¹ to C³ are used assuring that recombination only takes place in the respective subclass. As explained above it is possible to either use different restriction enzymes or to assure by for example nucleotide manipulation that different cohesive ends are created in the respective subclasses.

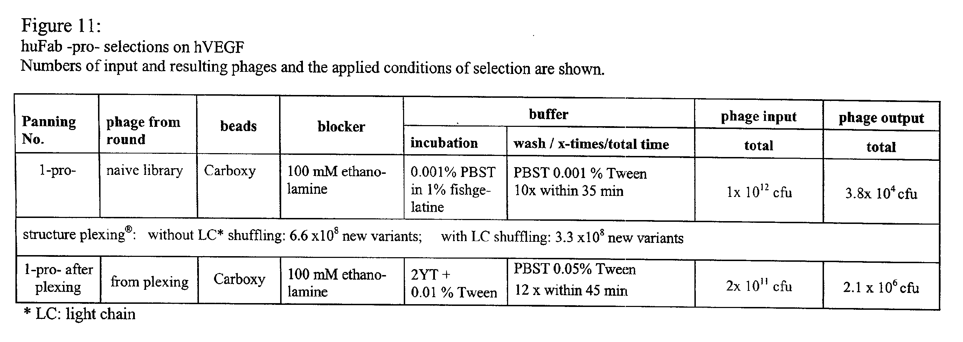

Fig. 11 shows the results of a completely human Fab library used for phage display-based selections on human VEGF (vascular endothelial growth factor) according to the invention (Example 3). Strongly ELISA-positive clones were recloned to produce soluble Fab fragments. These were tested for binding activity using surface plasmon resonance. Finally, purification and upconcentrating allowed for a kinetic analysis of soluble Fabs.

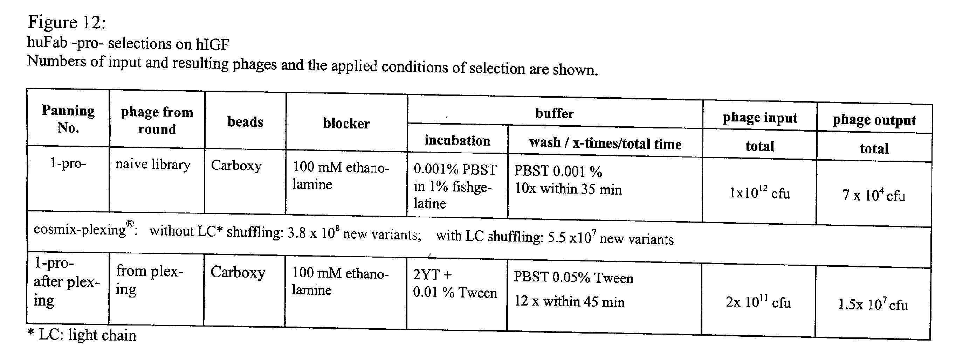

Fig. 12 shows the results of a completely human Fab library used for phage display-based selections on human IGF (insulin like growth factor) according to the invention (Example 4). Strongly ELISA-positive clones were recloned to produce soluble Fab fragments. These were tested for binding activity using surface plasmon resonance. Finally, purification and upconcentrating allowed for a kinetic analysis of soluble Fabs.

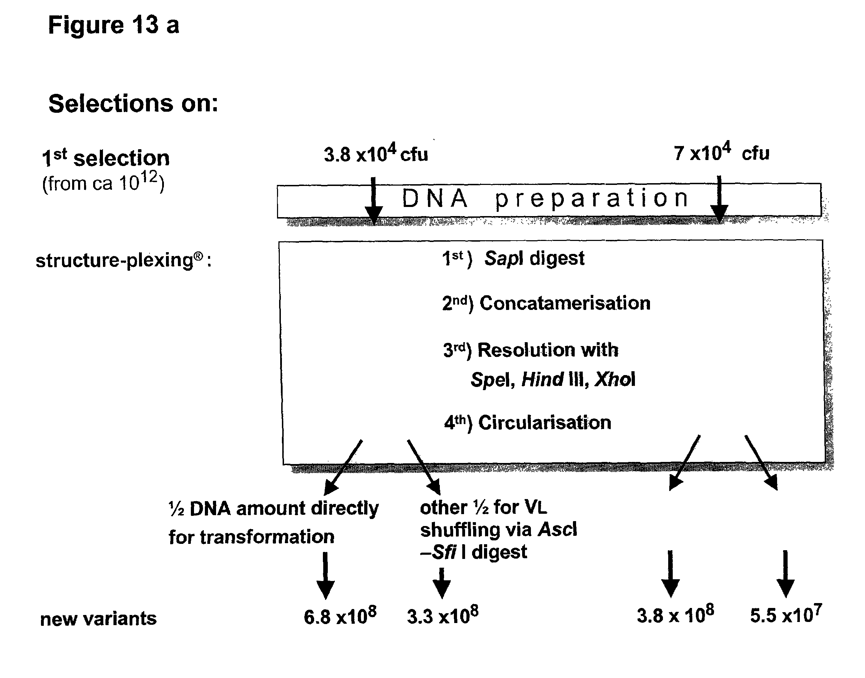

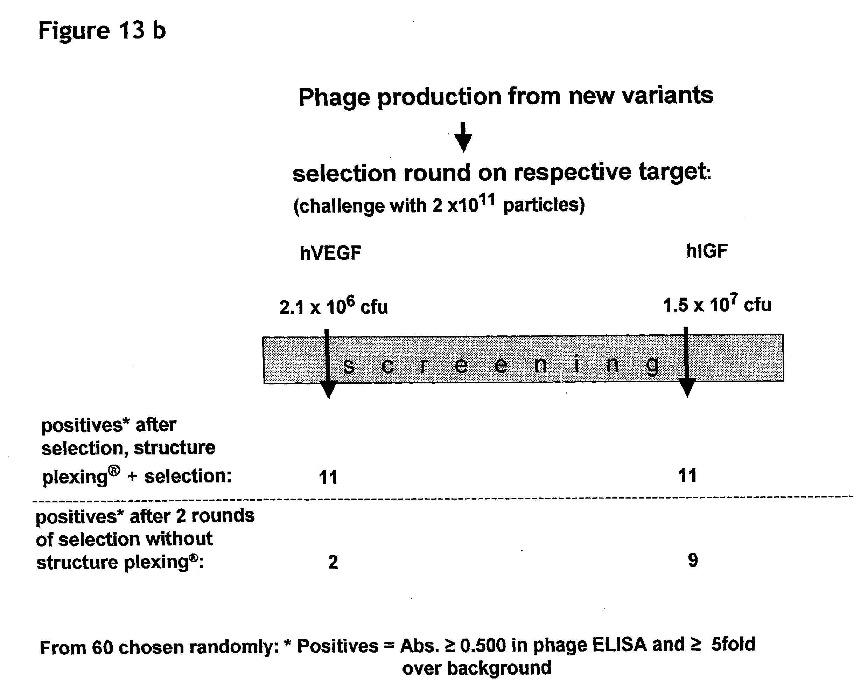

Fig. 13 a, b shows A schematic overview over the selection conducted according to examples 3 and 4;

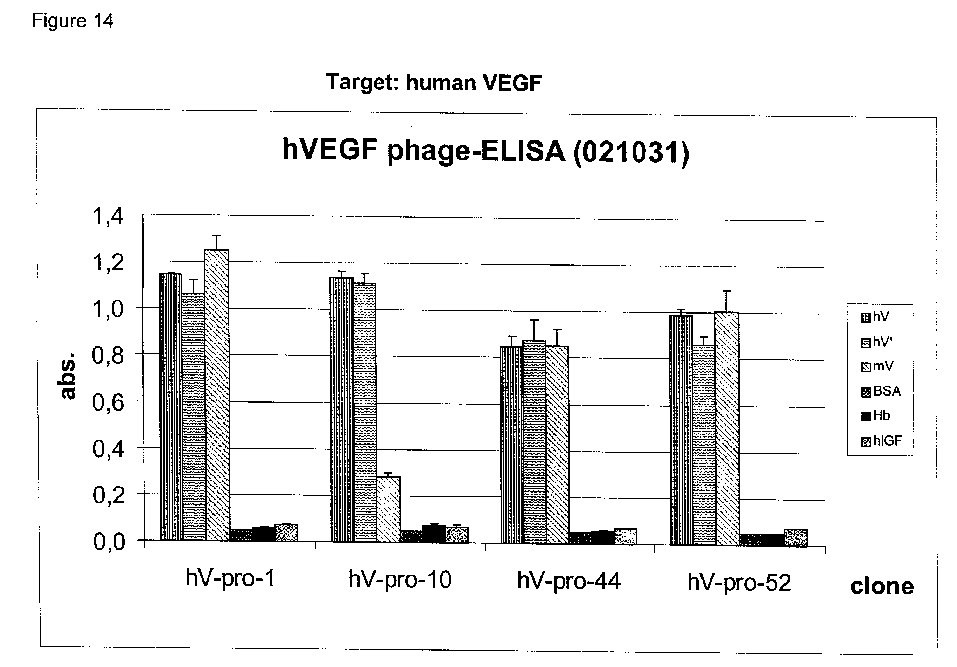

Figure 14 shows the Phage ELISA as described in example 3.

All antigens were immobilised at 100 ng/well. Detection was performed with a POD-conjugated anti-M13 monoclonal antibody. Absorption after staining with o-phenylene diamine was measured at 492 nm. hV: human VEGF; hV': another aliquot of human VEGF; mV: murine VEGF; BSA: bovine serum albumin; Hb: haemoglobin; hIGF: human IGF. Data are derived from double values.

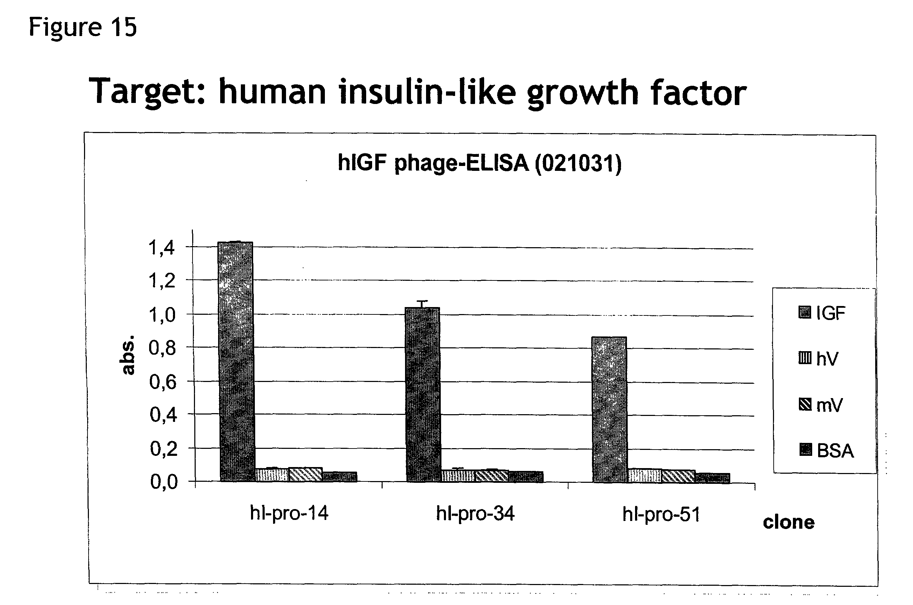

Figure 15 shows the Phage ELISA as described in example 4.
All antigens were immobilised at 100 ng/well. Detection was performed with a POD-conjugated anti-M13 monoclonal antibody. Absorption after staining with o-phenylene diamine was measured at 492 nm. hIGF: human IGF; hV: human VEGF; mV: murine VEGF; BSA: bovine serum albumin. Data are derived from double values.

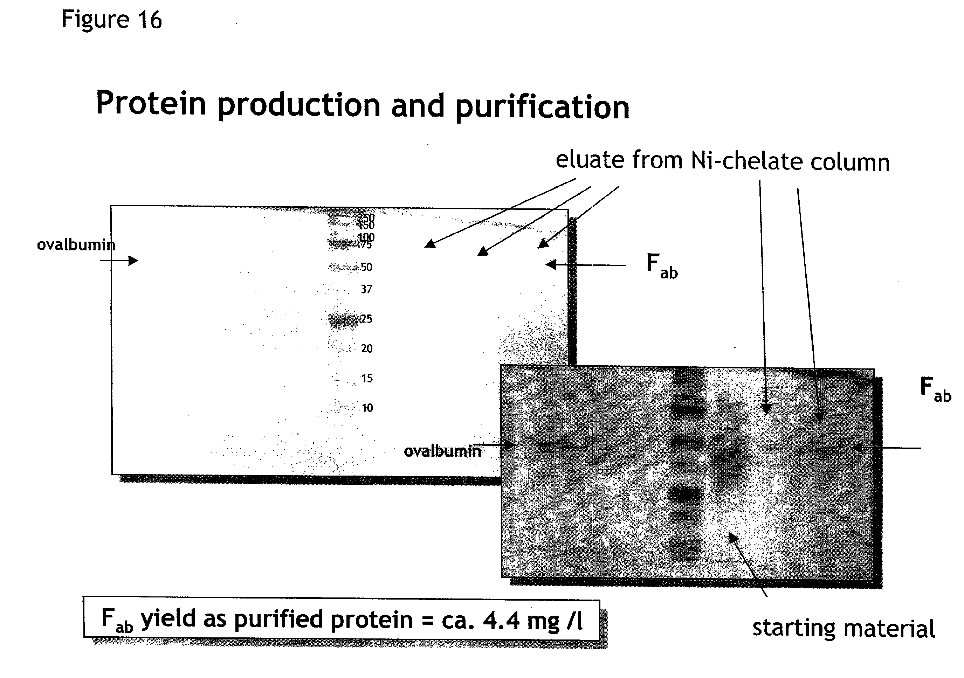

Figure 16 shows the production and purification of soluble Fab fragments. Amounts between 1 and 0.1 µg ovalbumin and 10 µl of starting material and of different eluate fractions were applied to SDS containing 10 % polyacrylamide gels to facilitate the estimation of sample amounts. The gels were stained with Coomassie colloidal staining (ICN).

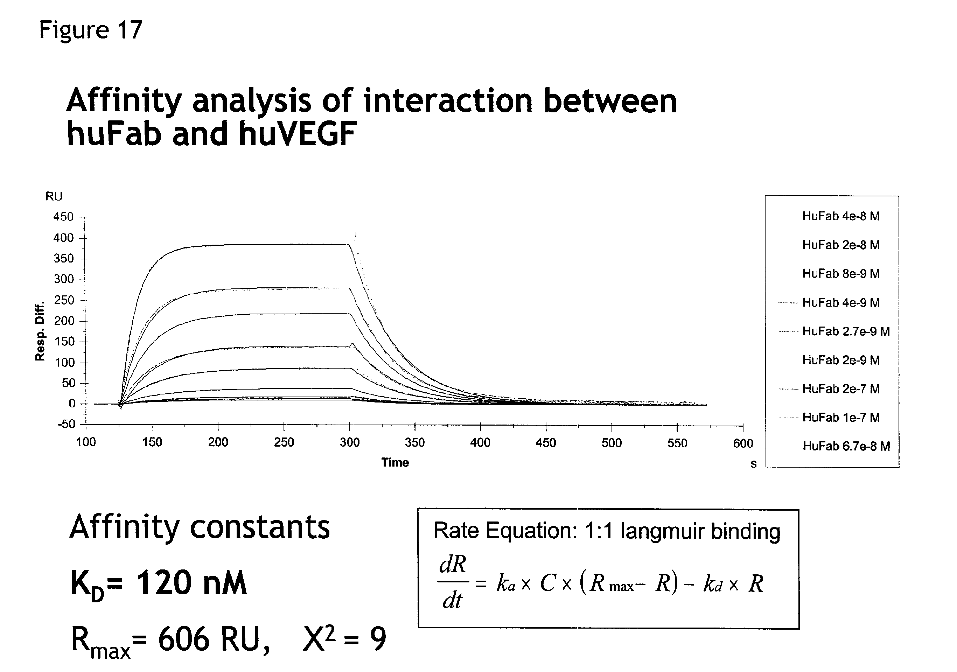

Figure 17 shows the kinetic analysis for hVEGF: Sensorgram curves obtained for a VEGF-specific clone applied in a concentration series to immoblized hVEGF fitted according to a 1:1 Langmuir binding.

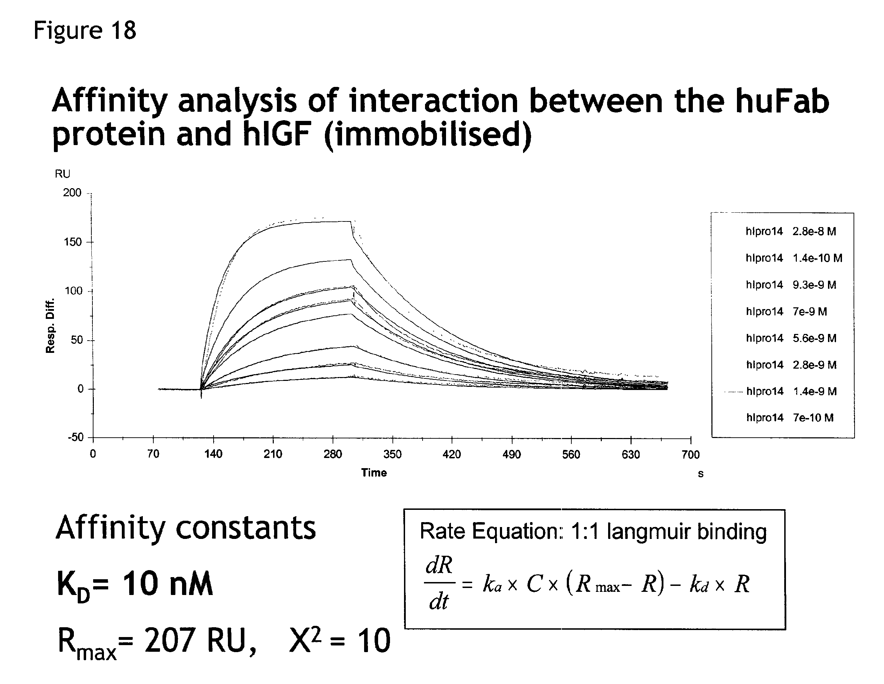

Figure 18 shows the kinetic analysis for hIGF: Sensorgram curves obtained for a hIGF-specific clone applied in a concentration series to immoblized hIGF fitted according to a 1:1 Langmuir binding.

### Example 1

### HUMAN NAIVE ANTIBODY LIBRARY FROM PERIPHERAL BLOOD

*Elucidated is the generation of an example* of a *human naive antibody library according to the present invention. Figs. 10a and 10b indicate which primers were used and where the generated fragments were introduced. Please note that the nucleotide residues at degenerated sites in the primers have the following meaning: R (purine; bases G and A)*, *Y (pyrimidine; C, T), M (amino; A,C), K (keto; T,G),* S *(strong; C,G), W (weak; A,T), B (not A), D (not C), H (not G) or V (not T). A site where no common feature is evident is designated N (any).*

### Background:

### 1. Preparation of White Blood Cells

Cells concentrated from half a litre human blood into a 60ml buffy coat were slowly poured into 95ml HBSS buffer (GIBCOBRL) supplemented with 5ml heparin (1000u/ml) and were then loaded onto 4x15ml Lymphoprep gradients (Nycomed-Life Technologies) in 50ml Falcon tubes. White blood cells were separated by centrifugation for 25min at 1,625rpm at room temperature. Upon centrifugation red blood cells migrated to the bottom of the gradient and white cells formed an interface between the lymphoprep and blood serum. Each of the interfaces was removed with a syringe and placed into new Falcon tubes and cells were pelleted by centrifugation for 10min at 1,600rpm , at room temperature. Pelleted cells were loosened by flicking the tubes, and were then lysed in 7.5ml RLT lysis buffer (RNeasy Kit, Qiagen), supplemented with 75µl of 14.4M β-mercaptoethanol. Genomic DNA was sheared by passing the lysate through a needle, for several times. Purification of the total cytoplasmic RNA was performed by following the procedure in manufacturer's manual (RNeasy Maxi Handbook, Qiagen).

### 2.Preparation of cDNA

On average 40µg cytoplasmic RNA were used for the first strand cDNA synthesis using the protocol as follows: 40µg RNA in 22.5µl were mixed with 2.5µl of 0.5mg/ml poly(dT)₁₆₋₁₈. The mixture was heated for 10min at 70°C and cooled on ice and was then spun for 5s at 5,000rpm. First strand cDNA synthesis cocktail was prepared in a final reaction volume of 50µl: 25µl RNA-poly(dT)₁₆₋₁₈, 2.5µl of 10mM dNTP, 10µl of 5x first strand buffer (GIBCO BRL), 5µl of 100mM DTT, and 5µl H₂O. The contents were mixed and prewarmed for 2min at 47°C and were then supplemented with 600u of RNase H Reverse Transcriptase (Superscript™II, GIBCOBRL). Reaction was performed for 1h at 47°C and terminated by heating for 15min at 70°C. Aqueous phase was transferred into new Eppendorf tubes after centrifugation for 30min at 15,000rpm and cDNA product was ethanol precipitated. The pellet was rinsed with 70% ethanol, air dried, and was then dissolved in 15µl DEPC-treated H₂O. 5µl of the samples were used as template in each of the primary PCR amplifications.

### 3. Primary PCR Amplification

Second strands of the antibody gene fragments were generated by PCR using a set of reverse oligoprimers specific for the heavy and light chain variable regions. In total, in heavy-variable region amplifications, 12; in kappa-variable region 8; and in lambda-variable region amplifications 12 reverse oligoprimers were used. In the amplification of gene fragments each primer within each of these three sets of primers were used separately and at differing concentrations. The final PCR amplifications included the said 12 V_{H}, 8 V?, and 12 V? primers were used with the respective forward primers to give 32 independent first strand PCRs for each of the 37 blood donors.

### 3.1 Final Concentrations of VH Primers in 100 µl PCR Reactions

Except oligoprimer VH8, heavy-chain variable fragments were used at 0.7nM final concentrations. VH8 was used at 1.4nM final concentration.

### 3.2 Final Concentrations of Vλ, Primers

Vλ1: 1.8nM;Vλ2,3,4,5,6,7,9, and 11: 0.9nM;Vλ8 and 12: 3.6nM; Vλ10: 4.5nM.

### 3.3 Final Concentrations of VK Primers

VK1,2,4,5,6, and 7: 200nM; VK3: 100nM; VK8: 50nM.
In each of the above three sets of reactions three different forward oligoprimers, each specific to one of the chains, were used at 200nM final concentrations. And amplification conditions were as follows: in a 1.5 mM final Mg+ concentration and using 1.5u *Taq* DNA polymerase (Amplitaq, Perkin Elmer) in a final reaction volume of 100µl, 5 min of initial denaturation were followed by 40 cycles of amplification: 1min denaturation at 94°C, 1min annealing at 55°C, and 1min extension at 72°C. Reactions were terminated with a 10min extension step at 72°C.

In each of the above three sets of reactions three different forward oligoprimers, each specific to one of the chains, were used at 200 nM final concentrations. And amplification conditions were as follows: 1.5 mM final Mg²⁺ concentration, 1.5 u *Taq* DNA polymerase (Amplitaq, Perkin Elmer) in a final reaction volume of 100??l, 5 min initial denaturation followed by 40 cycles of amplification: 1 min denaturation at 94°C, 1 min annealing at 55°C, and 1 min extension at 72°C. The final termination was a 10 min extension step at 72°C.

### 4. Quantification and Storage of Primary Amplification Products

In total, 37 different cDNA preparations were made using 18.5L human blood from 37 donors. In V_{H} gene fragment amplifications 32-36ml of PCR reaction pool, 30 (RNA samples)x12 (reverse oligoprimers)x100µl (each reaction volume), were obtained. Similarly, in kappa-chain amplifications approximately a reaction pool of 20ml, and in lambda-chain amplifications approximately 32ml were prepared. For a given chain, each of the reverse oligoprimer amplifications, from different RNA samples, were mixed together. These individual pools were quantified by agarose gel electrophoresis after phenol/chloroform purification and ethanol precipitation. Based on this type of quantification, for V_{H} gene fragments approximately 8µg DNA per reverse oligoprimer pool, and therefore, in total, 8x12=96 µg DNA were obtained. Similarly, for lambda-chain gene fragments approximately 150 µg, and for kappa-gene fragments approximately 200µg DNA were recovered. Individual reverse oligoprimer amplification products were dissolved in 400µl sterile water, then divided in four aliquots, and stored at -20°C.

Human H-chain V-region back primers
HVH-1 : CAG CCA GCA ATG GCA CAG GTN CAG CTG GTR CAG TCT GG
HVH-2 : CAG CCA GCA ATG GCA CAG GTC CAG CTK GTR CAG TCT GGG G
HVH-3 : CAG CCA GCA ATG GCA CAG GTK CAG CTG GTG SAG TCT GGG
HVH-4 : CAG CCA GCA ATG GCA CAG GTC ACC TTG ARG GAG TCT GGT CC
HVH-5 : CAG CCA GCA ATG GCA CAG GTG CAG CTG GTG GAG WCT GG
HVH-6 : CAG CCA GCA ATG GCA CAG GTG CAG CTG GTG SAG TCY GG
HVH-7 : CAG CCA GCA ATG GCA CAG GTG CAG CTG CAG GAG TCG G
HVH-8 : CAG CCA GCA ATG GCA CAG GTG CAG CTG TTG SAG TCT G
HVH-9 : CAG CCA GCA ATG GCA CAG GTG CAG CTG GTG CAA TCT G
HVH-10: CAG CCA GCA ATG GCA CAG GTG CAG CTG CAG GAG TCC GG
HVH-11: CAG CCA GCA ATG GCA CAG GTG CAG CTA CAG CAG TGG G
HVH-12: CAG CCA GCA ATG GCA CAG GTA CAG CTG CAG CAG TCA G

The nucleotides in bold correspond to the 5'-sequences of the V_{H} region starting with the codon for amino acid number one. Primers are invariant for the first five nucleotides from position +1 of the V region. The first 15 nucleotides at the 5'-end of the primers correspond to the 3'-part of the pelB leader.

Human H-chain J-region forward primers:

HJH1: GGC TGA GGA GAC RGT GAC CAG GGT
HJH2: GGC TGA AGA GAC GGT GAC CAT TGT
HJH3: GGC TGA GGA GAC GGT GAC CGT GGT

The J-region primers consist of three individually synthesized oligonucleotides representing a total of 4 variants. All nucleotides are complementary to the J-region and the first codon at the 5'-end corresponds to amino acid residue 114 of C_{H}1.

Human κ-chain V-region back primers

HVK-1: G CCG GCC ATG GCC GAC ATC CAR WTG ACC CAG TCT CC
HVK-2: G CCG GCC ATG GCC GAC ATC CRG ATG ACC CAG TCT CCW TC
HVK-3: G CCG GCC ATG GCC GAC ATC GTG MTG ACC CAG TCT CC
HVK-4: G CCG GCC ATG GCC GAC ATC GTG TTG ACS CAG TCT CCR GG
HVK-5: G CCG GCC ATG GCC GAC ATC GTG ATG ACY CAG WCT CCA C
HVK-6: G CCG GCC ATG GCC GAC ATC GTG ATG AYR CAG TCT CCA GC
HVK-7: G CCG GCC ATG GCC GAC ATC GTG MTG ACW CAG TCT CCA GA
HVK-8: G CCG GCC ATG GCC GAC ATC GTA ATG ACA CAG TCT CCA CC

These primers consist of eight individually synthesized oligonucleotides representing 27 variants. Nucleotides in bold correspond to the 5'-sequence of the V_{κ} gene starting with the codon for amino acid number one.

Human κ-chain constant-region forward primer

HCK.For: GTC TCC TTC TCG AGG CGC GCC TCA CTA ACA CTC TCC CCT GTT GAA GCT

HCK.For is complementary to codons for the seven carboxy-terminal amino acid residues of the C_{K} domain, indicated by bold letters. The tandem stop codons and the *Ascl* site are underlined.

Human λ-chain V-region back primers

HVL-1: G CCG GCC ATG GCC CAG TCT GYC CTG ACT CAG CCT G
HVL-2: G CCG GCC ATG GCC CAG TCT GCC CTG ACT CAG CCT C
HVL-3: G CCG GCC ATG GCC CAG TCT GTG CTG ACT CAG CCG TC
HVL-4: G CCG GCC ATG GCC CAG TCT ATG CTG ACT CAG CCC CAC TC
HVL-5: G CCG GCC ATG GCC CAG TCT GTG CTG ACT CAG CCA CCC TC
HVL-6: G CCG GCC ATG GCC CAG TCT GAG CTG ACT CAG GAC CCT GC
HVL-7: G CCG GCC ATG GCC CAG TCT GAG GTG ACT CAG GAG CC
HVL-8: G CCG GCC ATG GCC CAG TCT GTG ATG ACY CAG TCT CMA
HVL-9: G CCG GCC ATG GCC CAG TCT GTG CTG ACT CAG CCA CC
HVL-10: G CCG GCC ATG GCC CAG TCT GTS BTG ACG CAG CCG CC
HVL-11: G CCG GCC ATG GCC CAG TCT CAG CTG ACG CAG CCT GC
HVL-12: G CCG GCC ATG GCC CAG TCT TTA YTG ACT CAA YCG CCC TC

These primers consist of 12 individually synthesized oligonucleotides representing 24 variants. Nucleotides in bold correspond to the 5'-sequences of the Vλ genes starting with the codon for amino acid number one.

Human λ-chain constant-region forward primer

HCL.For: GTC TCC TTC TCG AGG CGC GCC TCA CTA TGA ACA TTC YGT AGG GGC MAC

HCL.For contains four variants and is complementary to codons for the seven carboxy-terminal amino acid residues of the λ constant domain, indicated by bold letters. The tandem stop codons and the Ascl site are underlined.

### 5. Heavy chain V-region design and amplifiations of heavy and light chains

The heavy chain V-regions were set up to have the following features: During the process of structure-plexing, the HCDR3 can be shuffled against HCDR1 and HCDR2 regions. This shuffling in V_{H} is allowed in all three Kabat subgroups but the respective subgroup framework scaffold will be preserved, using subgroup specific restriction sites.

### 5.1 Human H chain V-region extension PCRs

The extension PCRs for variable heavy chains were used to insert these subgroup-specific restriction sites in the FR3 of Kabat subgroups V_{H} I, V_{H} II, and V_{H} III (cf. Fig 2). To obtain subgroup specific sites, the heavy chain library 5'-fragments (FR1-FR3) were supplied with 5' *Nhel* and 3' *Spel, HindIII, and Xho*l recognition sites for V_{H}I, V_{H}II, and V_{H}III, respectively. The heavy chain library 3'-fragments (FR3-FR4, incl. HCDR3) were supplied with *Spe*l, *Hind*III, *and Xho*l recognition sites at the 5' end, respectively, and an *Mlu*l site at the 3' ends of each subgroup. Inserted FR3 restriction sites do not alter the amino acid sequence of the respective FR3. For illustration see figures 9 and 10.

### 5.1.1 5' part of H chain V-region extension PCRs

3µl of the primary PCR products were used as template for the 5' fragments. All primers were used after phosphorylation by polynucleotide kinase (MBI Fermentas) to allow for a concatamerisation of the final PCR products and yield better endonucleolytic digestion efficiency. Amplification conditions were as follows: 2 mM Mg²⁺, 200 µM dNTPs, 1 u Vent DNA polymerase (NEBiolabs) in a final reaction volume of 50?µl, 2 min initial denaturation (95°C) followed by 20 cycles of amplification: 30 s denaturation at 95°C, 30 s annealing at 48°C, and 30 s extension at 72°C. Primer concentrations were 1 µM.

Human H-chain V-region back primer for the 5' moiety:

HFR1Nhe+ : 5' CCTTCCTTGCTAGCAGCACAGCCAGCAATGGCACAG 3'

Human H-chain V-region forward primers for the 5' moiety:

H1FR3Spe-: 5' AGAAGGAAACTAGTGTCCGCGGTAATGGTG 3'
H4FR3Hind-: 5' TATAATAAAAGCTTCAGGGAGAACTGG 3'
H3FR3Xho-: 5' TATAATAACTCGAGAGATGGTGAATCGGCC 3'

The recognition sites are underlined. H1FR3Spe- was used to prime on primary PCR products obtained with primers HVH1, 2, 3, and 6. H4FR3Hind- was used to prime on primary PCR products obtained with primers HVH4, 7, 10, 11, and 12. H3FR3Xho- was used to prime on primary PCR products obtained with primers HVH3, 5, 8, and 9.

### 5.1.2 3' part of H chain V-region extension PCRs

2-4 µl of the primary PCR products were used as template for the 3' fragments. All primers were used after phosphorylation by polynucleotide kinase (MBI Fermentas) to allow for a concatamerisation of the final PCR products and yield better endonucleolytic digestion efficiency. Amplification conditions were as follows: 2 mM Mg²⁺, 200 µM dNTPs, 1 u Vent DNA polymerase (NEBiolabs) in a final reaction volume of 50?µl, 2 min initial denaturation (95°C) followed by 15-20 cycles of amplification: 30 s denaturation at 95°C, 30 s annealing at 58°C for V_{H}I (54 and 55°C for V_{H}II and V_{H}III), and 30 s extension at 72°C. Primer concentrations were 1 µM.

Human H-chain V-region back primers for the 3' moiety:

FR3H1Spe+:   5' CCAACCAAACTAGTACRAGCACAGCCTACATGG 3'
FR3H4Hind+: 5' CCAACCAAAAGCTTAGCTCTGTGACCGCCGCR 3'
FR3H3Xho+:  5' CAACCAATCTCGAGAYAATKCCAAGAACWC 3'

The recognition sites are underlined. FR3H1Spe+ was used to prime on primary PCR products obtained with primers HVH1, 2, 3, and 6. FR3H4Hind+ was used to prime on primary PCR products obtained with primers HVH4, 7, 10, 11, and 12. FR3H3Xho+ was used to prime on primary PCR products obtained with primers HVH3, 5, 8, and 9.
Human H-chain V-region forward primer for the 3' moiety: JHMlu-: 5' TTGGTTACGCGTGGAGGCTGAAGAGACGGTGAC This primer was used on all primary PCR products. The recognition site is underlined.

### 5.2 Human kappa and lambda extension PCRs

Primary PCR products were used separated from each other in the extension Amplifications. 3-4 µl of the primary PCR products were used as template for the extension PCRs to provide the light chain library fragments with 5' *Sfi*l and 3' *Asc*l recognition sites comprising variable and constant domains (cf. Fig 10b). All primers were used after phosphorylation by polynucleotide kinase (MBI Fermentas) to allow for a concatamerisation of the final PCR products and yield better endonucleolytic digestion efficiency. Amplification conditions were as follows: 1.5 mM Mg²⁺, 200 µM dNTPs, 1 u Vent DNA polymerase (NEBiolabs) in a final reaction volume of 50 µl, 2 min initial denaturation followed by 15-20 cycles of amplification: 30 s denaturation at 95°C, 30 s annealing at 64°C for the kappa chains and 61°C for lambda chains, and 30-60 s extension at 72°C. Primer concentrations were 1 µM.

Human kappa and lambda L chain V-region back primers: 
VkSfi+: 5' CTTTCTTTGGCCCAGCCGGCCATGGCCGAC 3'
VlamSfi+: 5' TTTCTTTGGCCCAGCCGGCCATGGCCCAG 3'
 The *Sfil* site is underlined.

Human kappa and lambda L chain C-region forward primers: 
VkAsc-: 5' CTTTCTTTAGGCGCGCCTCACTAACAC 3'
VlamAsc-: 5' CTTTCTTTAGGCGCGCCTCACTATGAAC 3'
 The *Asc*l site is underlined.

### 5.3 PCR product preparation

V_{H} 5' moieties were pooled according to their subgroup and purified via 1.6 % agarose gel. DNA bands of correct size (260-280 bp) were cut out and purified by NucleoSpin Kit (Macherey Nagel). Purified fragments were concatamerised by running a ligase reaction with 1u T4-ligase per 10 µl reaction volume for at least 2 h at 10°C.
After heat inactivation, ligated fragments were cut with *Bcul* (isoschizomer of *Spel)* in the case of V_{H}I, *Hind*III (V_{H}II), and *Xho*I (V_{H}III), addionally with *Nhe*l for all three subgroups.
V_{H} 3' moieties were pooled and purified as the V_{H} 5' parts above. DNA bands of correct size (170 bp for V_{H}I and V_{H}III subgroup, 140 bp for V_{H}II subgroup) were cut out and purified as above. Purified V_{H} 3' fragments were concatamerised by running a ligase reaction with 1u T4-ligase per 10 µl reaction volume for at least 2 h at 10°C.
After heat inactivation, the ligated fragments were cut with *Bcul* (isoschizomer of *Spe*l) in the case of V_{H}I, *Hin*dIII (V_{H}II), and *Xho*l (V_{H}III), addionally with *Mlu*l for all three subgroups.
To remove the resulting end fragments the V_{H} 5' and V_{H} 3' fragments were again purified by NucleoSpin Kit (Macherey Nagel).
All kappa light chains and all lambda light chains were pooled, each, fragments of ca 690 bp separated over a 1.5 % agarose gel, GFX purified, and concatamerised by running a ligase reaction with 1u T4-ligase per 10 µl reaction volume for at least 2 h at 10°C.
The ligated DNA was subjected to endonucleolytic digestion with *Ascl.* After heat inactivating *Ascl,* kappa and lambda light chain DNA was *Sfi*l digested for at least 3 h. The fragments of correct size were separated by agarose gel electrophoresis (1 % agarose) and purification of the excised DNA fragments with GFX or NuecleoSpin kit (Amersham Pharmacia or Macherey Nagel).

### 6. Preparation of the phagemid vector, directed mutagenesis

### 6.1 Introduction of the Sapl site separating heavy and light chain

The underlying phagemid vector pFab74 was developed in the laboratory of Prof Dr J. Engberg, Royal Danish School of Pharmacy, Copenhagen, Denmark, following the expression vector pFab60 (Johansen et al., 1995). Figure 2 depicts the Fab expression cassette and the cloning sites of the resulting vector pHuFab. To enable the process of plexing a *Sapl* site was introduced between constant light and variable heavy chain via PCR. *Sapl* is a type Its restriction endonuclease. Thus, the resulting non-palindromic overhangs could be designed to differ between V_{H}l, V_{H}ll, and V_{H}lll, ensuring that a preselected light chain pairing functionally with a certain heavy chain (from one of the three subgroups) will again find heavy chains of the same subgroup. Introduction of *Sapl* was carried out using the primers AscSap⁺, AscSapVH3⁺, or AscSapVH4⁺, respectively, together with the reverse primer 3'vNhe-.

AscSap+ 5′ TGATGAGGCGCGCCGCTCTTCGAGAAAAGGAGACAGTC 3′
AscSapVH3+ 5′ TGATGAGGCGCGCCGCTCTTCGGGGAAAGGAGACAGTC 3′
AscSapVH4+ 5′ TGATGAGGCGCGCCGCTCTTCGTAAAAAGGAGACAGTC 3′
3′vNhe- 5′ CACCCAGTTCATCCAAGAGG 3′
HUI SfiI+ 5′ TGGATTGTTATTACTCGCGG 3′

The *Sap*l recognition site is underlined, the overhangs resulting from endonucleolytic cleavage of the V_{H} subgroup-encoding DNA is in bold letters.

The above primers were used at 1 µM, Vent exo DNA polymerase (NEB) at 0.8 u in a total of 50 µl. Annealing was allowed at 56°C for 30 s, elongation at 72°C for 30 s in 35 cycles. The resulting fragments of ca. 210 bp were restriction digested with *Nhe*l, gel separated, and the 90 bp-fragments were purified by NucleoSpin Kit (Macherey Nagel) and used as reverse primers together with the primer HUI Sfil+ in the following PCRs for each intended V_{H} subgroup separately. Primers ca. 1 µM, Vent exo⁻ DNA polymerase (NEB) at 0.8 u in a total of 50 µl. Annealing was allowed at 55°C for 30 s, elongation at 72°C for 60 s in 35 cycles. The resulting fragments of ca. 800 bp were *Sfil*-digested, GFX-purified and ligated into the vector which had been prepared by *Sfi*l and *Nhe*l restriction digests and gel purification.
After these steps the three resulting vectors contained the desired Sapl-recognition site followed by a V_{H}subgroup specific cut site, repectively.

### 6.2 Introduction of the V_{H}-subgroup specific restriction sites into the V_{H} sequences

The required V_{H}-subgroup specific restriction sites were obtained by the following steps. 5' V_{H}-fragments of each subgroup were concatamerised by ligation at 10°C for at least 2 h. After heat inactivation the DNA were cut by *Nhe*l, after this by *Bcu*l, *Hind*III, or *Xho*l for the V_{H}I, II, and III subgroup, respectively. The 3'V_{H} fragments of each subgroup were concatamerised as well, then digested by *Bcu*l, *Hind*III, or *Xho*l. The digested 5' and 3' V_{H} fragments were purified with a NucleoSpin Kit (Macherey Nagel), then ligated to give full length V_{H} fragments (5'+3'). Fragments of the correct size (ca 400 bp) were excised from an agarose gel, purified by GFX (Amersham Pharmacia), digested with *Mlu*l, purified again over spin columns, and finally ligated into the corresponding vectors (see step 6.1) prepared after excision of an *Nhe*l-*Mlu*l fragment.

### 7. Insertion of heavy chains into the prepared vectors

The vectors prepared for subgroups V_{H}I, V_{H}II, and V_{H}III now contained the subgroup-specific cloning sites *Spe*l, *Hin*dIII, and *Xho*l, respectively. These vectors were prepared further by digestion with *Nhe*l, then *Bcu*l (isoschizomer of *Spe*l), *Hin*dIII or *Xho*l each for at least 1 h at 37°C. The obtained linear vector fragments were or were not subjected to dephosphorylation (0.1 u calf intestine alkaline phosphatase; MBI Fermentas per µg DNA, 37°C for 30 min). The fragments were separated on 0.6 % agarose gels, excised, and purified with GFX columns.
1-2 µg vector fragments were ligated to a 3 molar excess of 5' V_{H}-fragments overnight at ca. 10°C. These 50-100 µl reactions contained 0.5-1 Weiss unit T4-ligase (MBI) per 10 µl reaction volume. The reactions were stopped and purified with GFX columns. The circularized DNA was transformed into *E. coli* JM101, plated on large 2YT agar plates (including 200µg/ml ampicillin, 20 µg/ml tetracyclin, and 0.4 % glucose) and grown overnight at 30°C.
The obtained 5'V_{H}-fragment libraries for subgroups V_{H}I, V_{H}II, and V_{H}lll were harvested from the plates and parts were used to inoculate maxi-cultures. The DNA of these cultures were prepared after a few hours growth at 30°C following the manufacturer's protocol (Macherey Nagel), then digested with *Mlu*l and *Bcu*l, *Hin*dlll, or *Xho*l, each for at least 2 h at 37°C. The 5' -fragment library vectors were purified after agarose gel electrophoresis with GFX columns. The following overnight ligations with the pre-digested 3' V_{H}-fragments were carried out under the same conditions as described for the 5'fragment-part of the V_{H}-library ligations were purified and used for transformations in *E. coli* JM101 as above. The resulting V_{H}-library clones were used to prepare DNA from maxi-cultures and to store bacteria as gycerol stocks at -80°C.

### 8. Cloning of light chains and insertion into the vector

The V_{H}-library vector obtained in step 7 was subjected to *Sfil* endonucleotlytic restriction, after this, to *Asc*l restriction. The open library vector was separated over a 0.6 % agarose gel and purified with GFX columns. 1-2 µg of prepared vector was ligated with a 3 to 6fold molar excess of *Sfi*l/*Asc*l-digested kappa or lambda chain inserts overnight at ca. 10°C. These 40-120 µl reactions contained 0.5-1 Weiss unit T4-ligase (MBI) per 10 µl reaction volume. The reactions were stopped and purified with GFX columns. Circularised DNA was transformed as described in step 7.

### 9. Origin of restriction endonucleases

The restriciton endonucleases *Bcu*I, *Hin*dIII, and *Mlu*I, *Nhe*I, and *Xho*I were purchased from MBI Fermentas, Germany; the enzymes Sfil, *Sapl, Ascl* were products of New England Biolabs, Germany.

### 10. Production of packaged phagemids

The frozen glycerol stocks for the V_{H} subgroup I, II, and III each paired with kappa and lambda light chains were used to inoculate 200 ml 2YT, 600µg/ml ampicillin, 0.4 % glucose. After growth and infection for 1 h at 37°C, bacteria were pelleted by centrifugation at 2000 xg for 10 min, then resuspended in 200ml 2YT, 600µg/ml ampicillin, 0.5 mM-IPTG and at least 10¹¹ helper phages M13K07. Infection was allowed for 30 min at 37°C. Packaged phagemids were produced for 10-11 h at 25-30°C. The phages were purified by a two-fold PEG/NaCl precipitation.

### Example 3

### Methods:

As the methods for example 1 and 2 were completely identical the following list of methods applies to both examples.

### 1. Phage display selection:

Purified recombinant VEGF or IGF was coupled to EDC-/NHS activated paramagnetic carboxylic beads (Dynal, Oslo, Norway) at 3 µg protein per 7x10⁷ beads in 10 mM sodium acetate, pH 4.5 . Still reactive carboxylic groups were inactvated by 100 mM ethanolamine at room temperature. After washing of the beads about 1x10¹² Fab displaying phages from the naive library were incubated with the immobilized target in 1 ml PBS including 1 % fish gelatine for 1 h. Washing was done 10x with PBST (0.001 % Tween 20) for a total of 35 min. Binding phages were eluted with 0.2 M glycine buffer, pH 2.2, then neutralized with 1 M Tris, pH 8.0. Eluted phages were used to infect exponentially growing *E. coli* TOP10 F'. Bacterial growth was allowed on large 2YT agar plates (containing ampicillin, tetracycline and glucose) for about 24 h at 32°C. Colonies were scraped off the plate. For details concerning the obtained number of eluted clones cf. table 1.
After the first round of selection, harvested bacteria were used to start a 100 ml bacterial culture to produce phagemid DNA in preparative amounts. After a few hours of growth bacteria were pelleted and subjected to DNA preparation according to the manufacturer (Macherey Nagel, Düren, Germany). This DNA was subjected to structure plexing®.

### 2. Structure plexing®:

Briefly, the structure plexing® of DNA encoding Fabs from the first round of selection was carried out as outlined in figure 1: *Sap*l digest: About 10 µg phagemid DNA was linearised at high concentration (between 0.2 and 1 µg DNA/µl) by *Sap*l according to the manufacturer (NEB, Frankfurt, Germany). After completion, the endonuclease was heat inactivated. Concatamerisation: Linearised DNA was ligated to concatamers using T4-DNA ligase (MBI Fermentas, St. Leon-Rot, Germany) for a few hours at 10°C. After completion, the ligase was heat inactivated. Resolution: Ligated DNA was digested with *Bcu*l, *Hind* III, and *Xho*l roughly providing the conditions suggested by the supplier (MBI Fermentas). Again, the reaction was terminated by heat treatment. Circularisation: Appropriate DNA dilutions were determined emperically in test ligations and used to circularise the resolved concatamer DNA in an overnight ligation at 10°C. Reaction volumes were decreased by lyophilisation. The resulting circular DNA was either used for a) purification via GFX columns (Amersham Lifesciences, Freiburg, Germany) or for a further step b) light chain shuffling: An appropriate amount of *Asc*l was used to linearise the circular DNA. After completion, the enzyme was heat inactivated, followed by an *Sfil* digest. Both enzymes were used following the supplier's instructions (NEB). To inactivate Sfil the reaction was purified via GFX columns, then ligated for a few hours at 10°C. This ligation was again GFX-purified. Both final purified DNAs (a and b) were used to transform
*E. coli* TOP10 F' by electroporation. Bacterial growth was allowed as described under 1. (phage display selection). Harvested bacteria were used to inoculate some 500 ml of 2YT including ampicillin. Helper phage infection was carried out after 1 h growth at 37°C. Phage production was allowed overnight at 25-28°C. To obtain purified and concentrated packaged phagemids the overnight cultures were pelleted, the supernatant was used for a double precipitation of phages in PEG/NaCl. Resulting packaged phagemids were used in a further round of selection. This selection followed the principles as described under 1. For variations please see table 1.

### 3. Screening of selected clones:

60 clones from round 1 after plexing were picked randomly from agar plates and grown for a few hours at 37°C in 96 well microtiter plates using 2YT (incl. ampicillin and glucose). After helper-phage infection phage production was allowed overnight at 30°C. Bacteria were pelleted, the supernatant was used in a screening ELISA. 100 ng human VEGF per well was coated o/n on polystyrene high binding plates (Corning, Amsterdam, Netherlands) at 8°C. After blocking the wells with BSA phage containing supernatants were incubated 1:1 with BSA for 1 h. Detection was carried out with a POD-conjugated anti M13K07 monoclonal antibody (Amersham Lifesciences). For staining o-phenylene-diamine was used in citrate buffer and absorption was measured at 492 nm. Results of the screening are roughly reflected in figure 1.

### 4. Phage ELISA:

More detailed phage ELISA were carried out with packaged phagemids which had been purified and concentrated by double precipitation in PEG/NaCl. Human and murine VEGF and irrelevant antigens had been coated as described above. Incubation of purified phages and detection were carried out as under 3 except that smaller volumes of phages were applied.

### 5. Production of soluble Fab fragments:

To produce soluble Fabs the phagemid vector of ELISA positive clones was *Eag*I digested (NEB) to remove the pill gene of M13K07. The vector was excised from a gel, purified and recircularised by ligation. In case of clones containing an *Eag*I recognition site in their variable sequence, the complete Fab encoding cassette was separated from the vector by sequential restrictions with *Not* I (MBI Fermentas) then *Sfi*I (NEB) and electrophoresis. The cassette was excised from the gel, purified via GFX columns and ligated with a vector lacking a Fab cassette and a pill gene. For both recloning approaches, inactivated ligations were used for the transformation of *E. coli* TOP10 F'. Soluble Fab fragments were obtained from the supernatant of IPTG-induced bacteria after growth for 6-8 h at 30°C.

### 6. Purification of Fab fragments:

Culture supernatant was dialysed against PBS or used undialysed for Nc-affinity chromato-graphy. Fab containing material was pumped through a HiTrap chelating column (Amersham Lifesciences) and eluted with a gradient of imidazol. Successful purification and concentration was analysed by dot blot experiments using a POD-labeled goat anti-human IgG (Fab) specific antibody and PAGE analyses (see Fig. 4). A series of different ovalbumin amounts were used to estimate the amount of Fab fragment after purification.

### 7. Kinetic analyses:

Technical procedure of kinetic analysis has been carried out as previously described by Karlsson et al. (1991) and according to BIACORE manuals (BIAapplications Handbook and Instrument Handbook, Biacore AB, Uppsala, Sweden). Data were analyzed by application of standard software package provided by BIACORE company (BIAevaluation version 3).
Brief description of analysis to obtain kinetic parameters for the huFab - antigen interaction:
BIACORE system for measuring molecular interactions has been used to study the kinetics of interaction between huFab and humanIGF or humanVEGF respectively. BIACORE uses a microfluidic unit in contact with a sensor surface for surface plasmon resonance detection. Antigen (humanlGF/humanVEGF) was immobilized in a dextran matrix attached to the sensor surface. The interaction of matrix bound antigen with the corresponding huFab in solution is monitored label free in real time. The interaction is displayed as a plot of response units versus time called sensorgram. Target proteins were immobilized with standard BIACORE amine coupling according to BIACORE manuals. To study the interactions huVEGF was immobilized for separate experimental approaches at a response level of 312 RU (response units) or 106 RU. Human IGF was immobilized at a level of 105 RU. Concentration series of huFabs in the range of 10⁻⁷ M to 10⁻⁹ M were injected in the microfluidic unit to monitor the interaction with the respective immobilized target. Data had been corrected for bulk effects and instrumental interference by subtracting sensorgrams from a reference cell. Subsequently data were analyzed with BIAevaluation software. Affinity constants were obtained by fitting experimental sensorgram curves to a rate equation for a 1:1 interaction between analyte (huFab) and immobilized ligand.

Karlsson R, Michaelsson A, Mattsson L. 1991
Kinetic analysis of monoclonal antibody-antigen interactions with a new biosensor based analytical system.
J Immunol Methods. 145: 229_40.

### Results:

Using the complete Human Fab-library highly specific phage could be isolated after two rounds of selection. VEGF-selected Fab displaying phages showed VEGF specificity in ELISA experiments compared to different control antigens(Figure 2). hIGF-selected phages proved to be specific for hIGF compared to controls (Figure 3). Recloning into soluble Fab format allowed kinetic analysis of soluble Fab fragments. PSR-experiments resulted in the following apparent dissociation constants (Figure 5-6).

### Reference list:

[1] A. K. Aggarwal, D. A. Wah, Curr. Op. Struct. Biol., 1998, 8, 19-25.
[2] P. S. Andersen, A. Stryhn, B. E. Hansen, L. Fugger, J. Engberg, S. Buus, Proc. Natl. Acad. Sci. USA, 1996, 93, 1820-1824.
[3] R. Carlsson, C. Furebring, A.-C. Malmborg-Hager, C. Borrebaeck (Bioinvent International AB) WO 02/48351A2, 2000.
[4] C. Chothia, A. M. Lesk, E. Gherardi, I. M. Tomlinson, G. Walter, J. D. Marks, M. B. Llewelyn, G. Winter, J. Mol. Biol., 1992, 227, 799-817.
[5] J. Collins, Ann. Rep. Combinat. Chem. Molec. Divers., 1997, 1, 210-262.
[6] A. Crameri, W. P. C. Stemmer, BioTechniques, 1995, 18.
[7] A. Crameri, E. A. Whitehorn, E. Tate, W. P. C. Stemmer, Nature Biotechnology, 1996, 14, 315-319.
[8] A. Crameri, S.-A. Raillard, E. Bermudez, W. P. C. Stemmer, Nature(Lond.), 1998, 391, 288-92.
[9] K. Deshayes, M. L. Schaffer, N. J. Skelton, G. R. Nakamura, S. Kadkhodayan, S. S. Sidhu, Chemistry & Biology, 2002, 9, 495-505.
[10] I. Fisch, R. E. Kontermann, R. Finnern, O. Hartley, A. S. Soler-Gonzalez, A. D. Griffiths, G. Winter, Proc. Natl. Acad. Sci. USA, 1996, 93, 7761-7766.
[11] H. R. Hoogenboom, G. Winter, J. Mol. Biol., 1992, 227, 381-388.
[12] H. R. Hoogenboom, A. D. Griffiths, K. S. Johnson, D. J. Chiswell, P. Hudson, G. Winter, Nucleic Acids Res., 1991, 19, 4133-4137.
[13] H. R. J. M. Hoogenboom (Target Quest B.V., Maastricht(NL)) EP 1054018A1, 1999.
[14] J. M. Joern, P. Meinhold, F. H. Arnold, J. Molec. Biol., 2002, 316, 634-656.
[15] J. A. Kolkman, W. P. C. Stemmer, Nature Biotechnol., 2001, 19, 354-359.
[16] R. C. Ladner, TibTech, 1995, 13, 426-430.
[17] M Ohlin, E. Soderlind, R. Carlsson (Bioinvent International AB, Lund, Sweden) WO0175091, 2001.
[18] A. J. Podhajska, W. Szybalski, Gene, 1985, 40, 175-182.
[19] C. Schmidt-Dannert, D. Umeno, F. H. Arnold, Nat. Biotechnol., 2000, 18, 750-753.
[20] Z. Shao, H. Zhao, L. Giver, F. H. Arnold, Nucleic Acids Res., 1998, 681-683.
[21] M. D. Sheets, P. Amersdorfer, R. Finnern, P. Sargent, E. Lindqvist, R. Schier, G. Hemingsen, C. Wong, J. C. Gerhart, J. D. Marks, Proc. Natl.. Acad. Sci. USA, 1998, 95, 6157-6162.
[22] W. P. C. Stemmer, BioTechnology, 1995, 13, 549-553.
[23] W. P. C. Stemmer, A. Crameri, K. D. Ha, T. M. Brennan, H. L. Heyneker, Gene, 1995, 164, 49-53.
[24] A. Stryhn, P. S. Andersen, L. O. Pedersen, A. Svejgaard, A. Holm, C. J. Thorpe, L. Fugger, S. Buus, J. Engberg, Proc. Natl. Acad. Sci. USA, 1996, 93, 10338-10342.
[25] W. Szybalski, Gene, 1985, 40, 169-172.
[26] C. A. Voigt, C. Martinez, Z.-G. Wang, S. L. Mayo, F. H. Arnold, Nature Struct. Biol., 2002, 9.
[27] P. M. Kirkham, F. Mortari, J. A. Newton, H. W. Schroeder Jr. EMBO J., 1992, 11, 603-609.
[28] E. A. Kabat, T. T. Wu, H. M. Perry, K. S. Gottesman, C. Foeller, Sequences of proteins of immunological interest. 5th Edition, Public Health Services, NIH, USA; NIH Publ. No.: 91-3242.
[29] R. A. Katz, Annual Rev. Biophy. Biomol. Struct. , 1997,26,27-45.
[30] H. R. Hoogenboom et al., Immunotechnology, 1998, 4(1), 1-20.
[31] (EP 0349578A; EP 0527839A; EP 0589877A.
[32] D. J. Chiswell, J. McCafferty, Trends Biotechnol., 1992, 10, 80-84.
[33] B. J. Kay, G. Winter, J. McCafferty (Eds.) *Phage display of peptides and proteins: a laboratory manual* (1996) Academic Press, Inc ISBN 0-12-402380-0.

## Claims

1. A recombination process for recombining a population of heterologous polynucleotides comprising variant groups and/or regions, wherein recombination is performed within one group of polynucleotides and/or between defined regions of polynucleotides by using different restriction sites in different groups and/or regions of polynucleotides.

2. A recombination process according to claim 1,
- wherein the polynucleotides comprise at least one section defining a structural unit and at least one section defining an interstructural motif;
- wherein in the population at least two variant interstructural motifs are present;
- wherein variant interstructural motifs are recombined separately by using different restriction sites in different interstructural motifs.

3. A recombination process for recombining at least two polynucleotides comprising at least a structural unit and at least one interstructural motif, comprising
- cleaving the polynucleotides with at least one restriction enzyme creating a non-palindromic cohesive end thus creating a recombination site;
- wherein the recombination site is present in the interstructural motif which comprises a coding region of the polynucleotide;
- ligating the resulting mixture of fragments.

4. A recombination process according to at least one of the above claims, **characterised in that** polynucleotides are recombined wherein the structural unit of different polynucleotides are heterologous and wherein the structural units are optionally made of structural subunits which are connected by interstructural motifs.

5. A recombination process according to claim 3, wherein the structural units and/or the structural subunits are recombined.

6. A recombination process according to claim 2, 3 or 4, wherein the interstructural motif comprises a coding region of the polynucleotide, a non-coding region or a part of a vector sequence.

7. A recombination process according to at least one of the above claims , wherein at least one restriction enzyme is used which creates a non-palindromic cohesive end.

8. A recombination process according to at least one of the above claims, wherein the restriction endonuclease creating the non-palindromic cohesive end is a PRE.

9. A recombination process according to at least one of the above claims, wherein polynucleotides are recombined which are present in a vector.

10. A recombination process according to at least one of the above claims,
- wherein the polynucleotides are cleaved with at least one restriction enzyme creating non-palindromic cohesive ends,
- wherein the cleaved vectors are ligated at high DNA concentrations allowing the formation of concatemers,
- wherein the concatemers are resolved by cleavage with at least one further restriction enzyme
- wherein the vector constructs are recircularised by ligation.

11. A recombination process according to at least one of the above claims, wherein for different interstructural motifs different restriction enzymes are used thus creating unique sites for recombination.

12. A recombination process according to at least one of the above claims, wherein for different interstructural motifs the same restriction enzymes is used wherein the nucleotides are different at the cleavage site in different interstructural motifs thus creating unique restriction sites for recombination.

13. A recombination process according to at least one of the above claims, wherein a resolving enzyme is used which creates cohesive ends.

14. A recombination process according to at least one of the above claims, wherein a resolving enzyme is used which creates non-palindromic cohesive ends.

15. A recombination process according to at least one of the above claims, wherein the restriction site for the resolving enzyme is present in the interstructural motif.

16. A recombination process according to at least one of the above claims, wherein the binding sites for one or more restriction enzymes and/or the nucleotide variation at the cleavage site are introduced by site specific mutation at predefined positions.

17. A recombination process according to at least one of the above claims, wherein the polynucleotides are vector constructs comprising sequence sections defining at least two structural units.

18. A recombination process according to at least one of the above claims, wherein the polynucleotides comprise a variant gene family.

19. A recombination process according to at least one of the above claims,
- wherein the polynucleotides comprise a section encoding at least a variable heavy fragment of an antibody and/or a section encoding at least a variable light fragment of an antibody.

20. A recombination process according to at least one of the above claims,
wherein a population of polynucleotides is recombined wherein the polynucleotides encode at least the variable fragment of an antibody which defines the structural unit,
wherein the CDR regions of the variable fragment define the structural subunits that are recombined with each other and at least the FRs define the interstructural motifs,
wherein at least two different subclasses of the variable fragments are encoded by the polynucleotide population thus defining different groups,
wherein recombination is performed within each subclass separately by using for each subclass a different restriction site in the interstructural motif.

21. A process according to at least one of the above claims, wherein the variable heavy fragment comprises the VH and CH1 domains and the and variable light fragments comprise the VL and the CL domains.

22. A process according to at least one of the above claims, wherein the different framework subclasses present in the population of polynucleotides which are recombined are cleaved with different restriction enzymes creating cohesive ends.

23. A process according to at least one of the above claims, wherein the restriction site is present within the FR3.

24. A process according to at least one of the above claims, wherein the polynucleotides encode the variable heavy and the variable light fragment of an antibody.

25. A process according to at least one of the above claims, wherein at least one additional recombination site is located between the variable heavy and the variable light fragment which is used for shuffling the variable heavy fragment against the variable light fragment.

26. A process according to at least one of the above claims, wherein a restriction endonuclease is used for shuffling the variable heavy fragment against the variable light fragment, which creates non-palindromic cohesive ends.

27. A process according to at least one of the above claims, wherein the polynucleotides are vector constructs which comprise sections encoding the variable heavy and the variable light fragment of an antibody, wherein at least two different subclasses of the variable fragments (heavy and/or light fragment) are encoded by the polynucleotide population thus defining different groups,
- wherein the vectors are cleaved with at least one restriction enzyme creating a non-palindromic cohesive end at a restriction site present in-between the sections encoding the variable heavy and the variable light fragment of an antibody thus allowing recombination between the heavy and the light fragments;
- wherein upon usage of the at least one restriction enzyme a different cohesive end site is created in each group;
- wherein the fragments are ligated thus allowing the formation of concatemers containing only members of one group;
- wherein the concatemers are resolved by the use of at least one restriction enzyme at a restriction site present at an interstructural motif.

28. A process wherein, for resolving of the concatemers different restriction enzymes are used for different subclasses.

29. A process according to at least one of the above claims, wherein a phage and/or phagemid display vector is used as a vector.

30. A method for evolving a molecule by selection and recombination comprising a recombination process according to at least one of the above claims.

31. The method according to claim 30 comprising:
a.) creating a starting library by cloning the polynucleotides to be recombined into an expression vector if the polynucleotide itself is not a vector
b.) recombining the library members with a process according to at least one of the above claims 1 to 29
c.) selecting candidates with desired characteristics
d.) optionally performing further rounds of selection and recombination.

32. The method according to at least one of the above claims, wherein the library members respective the selected candidates are recombined with themselves or with other selected clones or members of the naive library.

33. The method according to at least one of the above claims, wherein the polynucleotides are expressed via a method which allows physical coupling of pheno- and genotype.

34. The method according to at least one of the above claims, wherein the recombined polynucleotides are expressed in a cell and selection is performed by screening or selecting for a particular phenotype amongst the clones.

35. The method according to at least one of the above claims, wherein the oligonucleotides are selected for physical properties.

36. The method according to at least one of the above claims, wherein a display library is created.

37. The method according to at least one of the above claims, wherein the selection step comprises selection for affinity to a defined target.

38. The method according to at least one of the above claims, wherein a phagemid library is created.

39. The method according to at least one of the above claims, wherein the polynucleotides are mutagenised.

40. The method according to at least one of the above claims, wherein further recombination processes are performed in addition to the recombination processes according to at least one of the above claims.

41. The method according to at least one of the above claims, wherein the further recombination processes are in vitro and/or in vivo recombination processes.

42. The method according to at least one of the above claims 31 to 41, wherein after step a.) and before step b.) a selection procedure is.

43. The method according to at least one of the above claims, wherein recombination is performed at a type IIs restriction site already present in the library.

44. A library containing heterologous polynucleotides comprising variant groups and/or variant regions within the polynucleotide, wherein the members of one group and/or the same regions have identical unique restriction sites which differ from the restriction sites present in other groups and/or regions.

45. A library according to claim 44, wherein the polynucleotides comprise at least one section defining a structural unit and at least one section defining an interstructural motif, wherein in the population at least two variant interstructural motifs are present.

46. A library according to claim 44 or 45, wherein the structural unit of different polynucleotides are heterologous and wherein the structural units are optionally made of structural subunits connected by interstructural motifs.

47. A library according to at least one of the above claims wherein the polynucleotides are or are present in a vector.

48. A library according to at least one of the above claims, wherein the interstructural motif comprises a coding region of the polynucleotide, a non-coding region or part of the vector sequence.

49. A library according to at least one of the above claims, wherein the unique restriction sites are located at the interstructural motifs.

50. A library according to at least one of the above claims, wherein at least one interstructural motif has a binding site for a PRE.

51. A library according to at least one of the above claims, wherein the polynucleotides are derived from nature and/or are at least partially synthetical polynucleotides sequences.

52. A library according to at least one of the above claims, wherein the polynucleotides comprise a variant gene family.

53. A library according to at least one of the above claims,
- wherein the polynucleotides comprise a section encoding at least a variable heavy fragment of an antibody and/or a section encoding at least a variable light fragment of an antibody.

54. A library according to at least one of the above claims,
wherein the polynucleotides encode at least the variable fragment of an antibody which defines the structural unit,
wherein the CDR regions of the variable fragment define the structural subunits and at least the FRs define the interstructural motifs,
wherein at least two different subclasses of the variable fragments are encoded by the polynucleotide population thus defining different groups,
wherein each subclass has a different restriction site in the interstructural motif.

55. A library according to at least one of the above claims, wherein the variable heavy fragment comprises the VH and CH1 domains and the and variable light fragments comprise the VL and the CL domains.

56. A library according to at least one of the above claims, wherein the restriction site for a resolving enzyme is present within the FR3 of the heavy chain.

57. A library according to on of the above claims, wherin the restriction site for a resolving enzyme is present within the FR3 the light chain.

58. A library according to at least one of the above claims, wherein the polynucleotides encode the variable heavy and the variable light fragment of an antibody.

59. A library according to at least one of the above claims, wherein at least one additional recombination site is located between the variable heavy and the variable light fragment which is used for shuffling the variable heavy fragment against the variable light fragment.

60. A library according to at least one of the above claims, wherein the polynucleotides are vector constructs which comprise sections encoding at least the variable heavy and the variable light fragment of an antibody, wherein at least two different subclasses of the variable fragments (heavy and/or light fragment) are encoded by the polynucleotide population thus defining different groups.

61. A library according to at least one of the above claims, wherein the vector is a phage or phagemid vector.

62. A process for producing a substance with special characteristics, comprising
- recombining and selecting a polynucleotide library for special characteristics according to at least one of the claims 44 to 60 according to a process according to claim 1 to 43,
- recovering at least one clone comprising the polynucleotide depicting the desired characteristic, amplifying said polynucleotide in vivo or in vitro
- manufacturing the substance which is either the expression product of the polynucleotide or comprises the latter.

63. Use of a molecule generated from a library according to claim 61 by a method or process according to at least one of the above claims for the treatment of diseases or for therapy.

64. Use of the products derived by the process of the present invention for deriving human therapeutic antibodies and antibody fragment conjugates.

65. Use of antibodies or antibody fragments generated from sequences which are selected from a library according to at least one of the above claims according to method or process according to at least one of the above claims, as diagnostics and/or therapeutic products for the treatment of diseases including immune dysregulation (including psoriasis), cancer, septic and toxic shock, blood haemostasis, nerve regeneration, pain, psychological disorders, appetite dysregulation, sexual dysfunction and wound healing or for vaccines.

66. Use of antibodies or antibody fragments generated from sequences which are selected from a library according to at least one of the above claims according to method or process according to at least one of the above claims for targeted delivery of pharmaceuticals to differentiated or neoplastic animal or human cells or as cosmeceutical.
